# FASCICULE DE BREVET EUROPEEN

(11) **EP 2 131 869 B1**
(45) Date de publication et mention de la délivrance du brevet: **17.10.2018**
(21) Numéro de dépôt: 08775556.7
(22) Date de dépôt: 14.02.2008
(51) Int. Cl.: A61K 49/00, A61K 9/107, B01F 17/00

(54) **EMULSIONS FLUORESCENTES POUR L'IMAGERIE OPTIQUE**
FLUORESZIERENDE EMULSIONEN ZUR OPTISCHEN ABBILDUNG
FLUORESCENT EMULSIONS FOR OPTICAL IMAGING

(30) Priorité: 14.02.2007 WO PCT/FR2007/000269
(43) Date de publication de la demande: 16.12.2009
(73) Titulaire: Commissariat à l'Énergie Atomique et aux Énergies Alternatives, 75015 Paris (FR); Centre National de la Recherche Scientifique, 75016 Paris (FR); Sorbonne Université, 75006 Paris (FR)
(72) Inventeur: GOUTAYER, Mathieu, F-35400 St Malo (FR); TEXIER-NOGUES, Isabelle, F-38000 Grenoble (FR); FATTACCIOLI, Jacques, F-20000 Ajaccio (FR); BIBETTE, Jérôme, F-75005 Paris (FR); DA SILVA, Anabela, 13004 Marseille (FR)
(74) Mandataire: Gevers & Orès
(86) Numéro de dépôt international: PCT/FR2008/000196
(87) Numéro de publication internationale: WO 2008/125747

(56) Documents cités:
- WO-A-98/48845
- WO-A-2008/102065
- US-A1- 2005 079 131
- US-B1- 6 559 183
- KALCHENKO VYACHESLAV ET AL: "Use of lipophilic near-infrared dye in whole-body optical imaging of hematopoietic cell homing" JOURNAL OF BIOMEDICAL OPTICS, vol. 11, no. 5, septembre 2006 (2006-09), page Article No.: 050507, XP002511213 ISSN: 1083-3668
- TEDESCO A C ET AL: "Binding and photophysical studies of biocompatible magnetic fluid in biological medium and development of magnetic nanoemulsion: A new candidate for cancer treatment" JOURNAL OF MAGNETISM AND MAGNETIC MATERIALS ELSEVIER NETHERLANDS, vol. 310, no. 2, mars 2007 (2007-03), pages 2838-2840, XP002447726 ISSN: 0304-8853

### SEQUENCE LISTING

<110> COMMISSARIAT A L'ENERGIE ATOMIQUE CENTRE NATIONAL DE LA RECHERCHE SCIENTIFIQUE UNIVERSITE PIERRE ET MARIE CURIE (PARIS 6) Goutayer, Mathieu Texier-Nogues, Isabelle Fattaccioli, Jacques Bibette, Jérôme DA SILVA, Anabela
<120> EMULSIONS FLUORESCENTES POUR L'IMAGERIE OPTIQUE
<130> S263pct181 EXT
<150> PCT/FR 2007/000269
   <151> 2007-02-14
<160> 1
<170> PatentIn version 3.3
<210> 1
   <211> 4
   <212> PRT
   <213> artificial sequence
<220>
   <223> cRGD peptide
<220>
   <221> misc_feature
   <223> c(RGDf[e-s-acetylthioacetyl])K
<400> 1
   Arg Gly Asp Lys
   1

## Description

La présente invention est relative à des sondes fluorescentes se présentant sous la forme de nano-émulsions utilisables dans le domaine de l'imagerie fonctionnelle *in vivo* non invasive.

*In vivo,* le développement récent des méthodes optiques ouvre de nouveaux horizons pour l'imagerie fonctionnelle. Il est maintenant possible de suivre en temps réel et de façon non invasive, le devenir de molécules luminescentes, leur biodistribution, d'établir un diagnostic et d'évaluer l'effet d'une thérapeutique grâce à ces molécules. L'imagerie optique présente un certain nombre d'avantages par rapport aux autres techniques d'imagerie fonctionnelle telles que l'imagerie par résonance magnétique (IRM), l'imagerie par tomographie d'émission de positons (TEP) et l'imagerie par émission monophotonique (SPECT) :
- elle évite la manipulation de molécules radioactives, écartant ainsi les contraintes et les risques qui y sont liés (radioprotection, gestion des déchets, source synchrotron pour les marqueurs TEP) ;
- elle ne nécessite pas de gros investissement d'instrumentation ;
- elle présente une bonne sensibilité par rapport à l'IRM, en terme de quantité de marqueur injectée.

Les applications envisagées à court terme pour ces techniques optiques d'imagerie fonctionnelles sont, dans le petit animal, l'aide à la découverte de biomarqueurs permettant de cibler des tumeurs ou la plaque d'athérome, la découverte et l'évaluation de nouvelles thérapeutiques, la diminution du nombre d'animaux sacrifiés dans les études de toxicité, puisque ces techniques permettent un suivi longitudinal. A très court terme, des applications chez l'homme sont envisagées en chirurgie intra-opératoire dans le but de permettre une meilleure délimitation des berges d'exérèse des tumeurs par exemple. D'autres applications à moyen terme sont explorées en dermatologie, comme outil de diagnostic du cancer du sein, ou par endoscopie du cancer de la prostate ou de polypes précancéreux du colon.

L'imagerie de fluorescence nécessite l'injection préalable d'une sonde ("optical probe" en anglais), équivalent du traceur en médecine nucléaire ou de l'agent de contraste en IRM et tomographie X. Cette sonde est en général un assemblage moléculaire constitué au minimum d'un label fluorescent qui correspond à l'entité responsable de l'absorption et de l'émission de la fluorescence. Ce label peut être par exemple un fluorophore organique, un complexe de lanthanide, ou bien encore un nanocristal semi-conducteur luminescent ("quantum dot" ou boîte quantique, tel que CdSe, CdTe, InP, Si...).

Ces sondes peuvent en outre comporter un ou plusieurs des éléments suivants :
a) un ligand biologique, permettant d'imager un processus biologique spécifique. Un tel ligand peut être :
   i) un ligand biologique de ciblage : c'est alors une entité biologique (anti-corps, peptide, saccharide,...) ou chimique (acide folique par exemple) qui permet une reconnaissance spécifique de certaines cellules (par exemple de cellules tumorales comme décrit par exemple dans l'article de S. Achilefu, Technology in Cancer Research & Treatment, 2004, 3, 393-408) ou de certains organes,
   ii) un ligand biologique marqueur d'une activité biologique donnée, par exemple d'une activité enzymatique. Par exemple, ces ligands biologiques seront un peptide clivable par une protéase donnée, à l'extrémité duquel sera greffé un inhibiteur de la fluorescence du label. Ce type de ligands permet d'imager spécifiquement l'activité enzymatique de la protéase ainsi que cela est reporté dans l'article de C.H. Tung, Biopolymers, 2004, 76, 391-403. Un autre exemple est constitué par un ligand biologique comportant un pont disulfure séparant le label d'un inhibiteur de sa fluorescence. Ce ligand biologique permet alors d'imager spécifiquement l'internalisation de la sonde dans une cellule comme décrit par exemple dans la demande de brevet français publiée sous le numéro FR 2 888 938 ;
b) un agent de furtivité : c'est une entité que l'on rajoute à la sonde afin de lui conférer une furtivité vis-à-vis du système immunitaire, d'augmenter son temps de circulation dans l'organisme, et de ralentir son élimination ;
c) un "vecteur d'assemblage" : c'est une entité qui peut permettre d'assembler le(s) label(s) fluorescent(s), et/ou le(s) ligand(s) biologique(s) de ciblage, et/ou le(s) agent(s) de furtivité, et/ou une ou d'autres fonctionnalités (délivrance de médicaments, autre modalité d'imagerie, fonction thérapeutique par exemple).

A partir de ce principe général, quatre grands types de sondes ont été décrits dans la littérature pour l'imagerie de fluorescence non invasive du petit animal.

Le premier grand type de sondes correspond aux sondes ayant pour label un (ou des) fluorophore(s) organique(s) greffés directement sur le ligand biologique. Si le premier fluorophore organique utilisé en imagerie de fluorescence, l'ICG ("Indo Cyanin Green"), a été employé très tôt "nu" (injection du fluorophore seul) pour imager la vascularisation et la circulation dans les vaisseaux sanguins, ces fluorophores organiques ont ensuite été greffés sur des protéines ou des anti-corps pour cibler différentes cellules. Cependant le couplage à ces grosses molécules peut présenter des inconvénients pour le ciblage et la pharmaco-cinétique, et c'est pourquoi plus récemment la fonctionnalisation de fluorophores par de petits peptides a été préférée. D'une manière générale, ce premier type de sondes possède les inconvénients suivants :
- un ligand biologique n'est marqué que par un faible nombre de labels fluorescents (typiquement 1 fluorophore pour un peptide, 3 à 4 pour un anti-corps). Pour augmenter le nombre de fluorophores, il faut sinon utiliser des édifices moléculaires vecteurs complexes (voir ci-après). Ce sont donc des sondes conduisant à de faibles signaux de fluorescence ;
- les fluorophores organiques utilisés, dont les longueurs d'onde d'absorption et d'émission doivent se situer dans le proche infra-rouge (entre 650 et 900 nm), fenêtre optique pour laquelle l'absorption et la diffusion de la lumière dans les tissus biologiques sont minimales, sont généralement sensibles au photo blanchiment, et notamment en tampon aqueux (perte de fluorescence au cours du temps). L'offre de fluorophores organiques greffables à des biomolécules et solubles en tampon aqueux dans ce domaine de longueur d'onde est de plus limitée (exemples : cyanines commercialisées par Amersham, série des produits vendus sous la dénomination commerciale Alexa® par Invitrogen), et à des coûts très élevés alors que de nombreux autres fluorophores pour le proche infrarouge bon marché sont disponibles chez d'autres fournisseurs, mais ne sont malheureusement pas utilisables pour cette application car ils sont non solubles en milieu aqueux ;
- les sondes doivent pouvoir être injectées par voie intraveineuse en quantité importante car seule une faible partie d'entre elles atteint leur cible. En effet les cinétiques de reconnaissance cible-ligand sont souvent lentes et entrent en compétition avec les processus d'élimination par l'organisme. L'ajout d'un agent de furtivité peut permettre d'améliorer ce point. Néanmoins, et c'est le quatrième inconvénient de ces sondes ;
- l'ajout d'un agent de furtivité (comme éventuellement l'augmentation du nombre de labels fluorescents par ligand biologique, ou l'ajout d'autres fonctionnalités comme la multimodalité ou la délivrance de médicaments) nécessite d'utiliser un "vecteur d'assemblage" pour assembler les différents éléments de la sonde. La synthèse de ces sondes devient alors beaucoup plus complexe.

Le deuxième grand type de sonde correspond aux sondes ayant pour label un nanocristal semi-conducteur luminescent ("quantum dot" ou boîte quantique, tel que CdSe, CdTe, InP, Si...), fonctionnalisé par un ligand biologique.

Les propriétés optiques (absorption, émission, rendement quantique de fluorescence) exceptionnelles de ces nanoparticules en ont fait ces dernières années des labels privilégiés pour l'imagerie non invasive du petit animal (X. Michalet et al., Science, 2005, 307, 538-544). Cependant, si aucune toxicité n'a été observée au cours de ces expérimentations, la présence de métaux lourds dans le coeur de ces nanocristaux demeure un frein important lorsque l'on envisage des applications cliniques chez l'homme (R. Hardman, Environmental Health Perspectives, 2006, 114, 165-172). Un autre des verrous actuels à l'utilisation de ces sondes pour l'imagerie non invasive est le contrôle de leurs propriétés d'interaction avec les tissus biologiques. En effet, ces propriétés sont très dépendantes de l'état de surface de ces nanoparticules (présence de chaînes de poléthylèneglycol (PEG)) et de leur taille.

Le troisième grand type de sondes correspond aux sondes ayant pour label des nanoparticules inorganiques d'oxydes, telles que par exemple des nanoparticules de silice, encapsulant et/ou sur lesquelles sont greffés des fluorophores organiques ou des chélates de lanthanides. Jusqu'à présent, peu de travaux ont mis en oeuvre ce type de sonde *in vivo,* hormis les travaux de l'équipe de P. Prasad en nanomédecine (I. Roy et al., PNAS, 2005, 102(2), 279-284) bien que la synthèse de cette sorte de labels soit abondamment décrite dans la littérature. Comme les nanocristaux semi-conducteurs décrits ci-dessus, ces nanoparticules inorganiques nécessitent une chimie de surface très poussée afin d'y greffer des agents de furtivité les rendant compatibles avec le vivant.

Enfin, le quatrième grand type de sondes correspond aux sondes ayant pour label des nanoparticules organiques encapsulant et/ou sur lesquelles sont greffés des fluorophores organiques ou des chélates de lanthanides. Parmi celles-ci, sont recensées :
- des nanosphères de polymère encapsulant des fluorophores organiques comme décrit par exemple dans l'article de V. Holzapfel et al., J. Phys.: Condens. Mater., 2006, 18, S2581-S2594 ;
- des polymersomes qui sont des assemblages de polymères synthétiques amphiphiles se présentant sous la forme de nanosphères constituées d'une double couche de polymères de façon analogue aux liposomes qui sont, quant à eux, constitués de lipides. Ces polymersomes peuvent être utilisés pour encapsuler des fluorophores organiques comme décrit par exemple dans la demande de brevet américain n° US 2005/0019265 ou dans l'article de P. Ghoroghchian et al., Proc. Natl. Acad. Sci. U. S. A., 2004, 102(8), 2922-2927.

Bien que ces systèmes nanoparticulaires soient organiques, ils possèdent un certain nombre d'inconvénients. Les polymersomes et les liposomes montrent des problèmes de stabilité chimique et physique. En ce qui concerne les nanosphères de polymères, leur cytotoxicité et leur synthèse chimique (problème de solvant résiduel) limitent leur utilisation. Même les polymères synthétiques, qui montrent une bonne biodégradabilité et une faible cytotoxicité *in vitro* tel que les polymères d'acide lactique (PLA), de poly(*β*-hydroxybutyrate) (PHB) ou encore le poly(lactide-co-glycolide), possèdent des effets cytotoxiques lorsqu'ils sont délivrés sous forme de nanoparticules.

Les sondes utilisées pour l'imagerie de fluorescence décrites jusqu'à présent dans la littérature présentent donc généralement un ou plusieurs des inconvénients suivants :
- une faible fluorescence par ligand biologique, surtout en milieu biologique,
- une tendance au photoblanchiment,
- une chimie d'assemblage ou de fonctionnalisation de surface complexe,
- une toxicité potentielle lorsqu'elles se présentent sous la forme de nanoparticules inorganiques, ce qui nécessiterait la mise en oeuvre d'une chimie de surface supplémentaire pour les rendre biocompatibles, rendant ainsi difficile leur utilisant pour l'imagerie humaine ;
- une complexité de synthèse dans la mesure ou les nanoparticules inorganiques sont difficiles à synthétiser, notamment en grandes quantités, et nécessitent l'emploi de solvants toxiques tels que le trioctylphosphine (TOP) et l'oxyde de trioctylphosphine (TOPO), et de matériaux dangereux (Cd(Me)₂,...). Les prix de revient de ces synthèses sont par ailleurs très élevés ;
- faible stabilité et synthèse peu reproductible des nanoparticules organiques (polymersomes) ;
- cytotoxicité des nanoparticules organiques de polymère et la synthèse à grande échelle pas encore maîtrisée ;
- une durée de vie de fluorescence trop courte empêchant leur utilisation pour l'imagerie de fluorescence résolue dans le temps.

L'utilisation de techniques de fluorescence résolue dans le temps présente de nombreux avantages par rapport aux méthodes stationnaires.

Premièrement, c'est la technique de prédilection lorsqu'il s'agit de travailler en mode fluorescent car le signal mesuré contient potentiellement toutes les informations sur la structure du milieu et sur les fluorophores. En effet, les techniques utilisant des signaux continus (CW : « continuous-wave ») sont basées sur la mesure simple de l'atténuation du signal incident. La détermination de la distribution de molécules fluorescentes se fait par mesure du signal de fluorescence, directement proportionnel à la concentration locale en molécules fluorescentes. Cette technique suppose donc la connaissance des propriétés optiques (d'absorption et de diffusion) du milieu diffusant environnant, et ne prend pas en compte les propriétés intrinsèques du fluorophore liées au temps de vie. L'utilisation de signaux résolus en temps (signaux modulés en amplitude au cours du temps ou impulsionnels) offre la possibilité à la fois de lever l'indétermination sur les propriétés optiques (de diffusion et d'absorption) du milieu - ce qui permet de mieux modéliser le phénomène physique de propagation lumineuse - et de se servir de l'information sur le temps de vie du fluorophore pour identifier sa position avec plus de précision.

Deuxièmement, pour la détection *in vivo* sur des cellules, des tissus biologiques, voire un animal ou chez l'homme, l'utilisation de techniques pulsées permet aussi de diminuer la dose globale d'irradiation appliquée, tout en ayant des puissances crêtes suffisantes pour une détection très sensible. Cependant, dans un milieu très diffusant ou auto-fluorescent, tels que le sont les tissus biologiques, la sensibilité de détection du signal spécifique des sondes fluorescentes peut être diminuée par l'auto-fluorescence ou la diffusion non spécifique du milieu. Le problème est que le milieu, même si ses propriétés d'auto-fluorescence intrinsèques sont faibles, est présent en quantité majoritaire par rapport au fluorophore à détecter. Quelle que soit la configuration expérimentale dans laquelle on se place (Imagerie de fluorescence en réflexion : FRI ou Imagerie de fluorescence par transillumination : TFI), il faut arriver à filtrer optiquement, mais aussi discriminer sur le signal collecté au niveau du détecteur, la fluorescence du fluorophore de l'auto-fluorescence et de la diffusion du signal d'excitation.

La durée de vie d'auto-fluorescence du milieu est généralement très courte (de l'ordre de 200 à 500 ps typiquement). Afin de discriminer au mieux les sondes fluorescentes de ce fond auto-fluorescent et de tirer tout le bénéfice des méthodes résolues en temps par rapport aux méthodes stationnaires, il est donc favorable d'utiliser des sondes fluorescentes très lumineuses et à durée de vie de fluorescence "longue", supérieure à 0.5 ns. D'un autre côté, l'utilisation de fluorophores à durée de vie trop longue ne permet pas d'enregistrer correctement un déclin du signal entre deux acquisitions. On n'a alors aucune information complémentaire par rapport à l'information obtenue avec une mesure utilisant une excitation continue. De plus, cela ne permet pas non plus au signal de revenir à zéro entre deux acquisitions. Les systèmes d'acquisition optique en pulsé utilisés dans le domaine clinique fonctionnent typiquement à 8 MHz. Il n'est pas possible de travailler au-delà de 10 MHz environ afin de ne pas brûler les tissus. Si on travaille à des cadences trop basses, on perd cependant en sensibilité du signal. Il faut donc pour tirer bénéfice des méthodes résolues en temps employer des fluorophores de durée de vie de fluorescence inférieure à 10-20 ns, pour que le signal soit revenu proche de zéro au cours d'une fenêtre d'acquisition de 100-125 ns (cadence de 8-10 MHz).

La gamme de durée de vie de fluorescence idéale pour l'imagerie de fluorescence résolue en temps est par conséquent comprise entre 0,5 ns et 10 ns. Or, pour les applications en milieu biologique ou en milieu turbide, travailler dans le domaine du proche infrarouge (640-900 nm), pour lequel la diffusion, ainsi que l'absorption de la lumière par les tissus ou l'auto-fluorescence des tissus sont réduites par rapport au domaine visible, est absolument essentiel. Il n'existe à l'heure actuelle pratiquement aucun fluorophore adéquat absorbant et émettant dans le domaine du proche infrarouge (640-900 nm) avec des durées de vie de fluorescence comprises entre 0.5 et 10 ns.

Les nano-émulsions, appelées quelques fois mini-émulsions, émulsions ultrafines ou encore émulsions submicroniques, sont des émulsions dont le diamètre est généralement compris entre 10 et 200 nm. Pour rappel, une émulsion est un mélange de deux substances liquides non miscibles constitué d'une phase continue et d'une phase dispersée. Une substance est dispersée dans la seconde substance (phase continue) sous forme de petites gouttelettes (phase dispersée). Le mélange reste stable grâce à l'action de molécules amphiphiles, appelées émulsifiants ou tensioactifs, qui se placent à l'interface entre les deux phases. Les émulsions sont des édifices supramoléculaires métastables. Ces structures sont à différencier des polymersomes et des micelles.

Les polymersomes (famille comprenant les liposomes) sont des vésicules de quelques dizaines à quelques milliers de nm de diamètre. Ces vésicules sont composées d'une ou de plusieurs bicouches de tensioactifs qui permet(tent) de séparer le milieu intravésiculaire du milieu extérieur, les deux milieux étant de même nature (aqueux).

Les micelles consistent en des agrégats de tensioactifs autoassemblés, de quelques nanomètres de diamètre. Les tensioactifs s'organisent de manière à orienter leur partie hydrophile vers l'extérieur (le solvant) et leurs chaînes hydrophobes vers le coeur de la micelle.

Des systèmes d'émulsions destinées à différentes applications dans le domaine du médical ont déjà été décrits dans la littérature :
- systèmes de nutrition parentérale qui sont des émulsions à base de lipides injectées par voie intraveineuse comme source d'acides gras essentiels, de vitamines lipophiles et d'énergie. Ces émulsions sont généralement constituées d'un mélange d'huiles d'origine naturelle (végétale ou de poisson) ;
- systèmes pour la délivrance de médicaments (*"Drug Delivery*") qui sont des émulsions de type huile dans eau étudiées et utilisées notamment pour l'encapsulation de principes actifs peu solubles dans l'eau. Ces émulsions ont comme avantage d'améliorer la biodisponibilité du principe actif qu'elles contiennent et de réduire ses effets secondaires, mais aussi d'augmenter sa stabilité en limitant son hydrolyse. Les émulsions sont utilisées pour 3 modes d'administration différents : oculaire / parentéral / oral. Pour limiter la métabolisation des émulsions administrées par voie parentérale, certaines formulations ont recours à l'utilisation de co-tensioactifs de furtivité, augmentant ainsi le temps de circulation des émulsions dans le système sanguin ;
- des systèmes renfermant des colorants utilisables comme marqueurs histologiques pour la résection de tumeurs cancéreuses. De telles émulsions sont par exemple décrites dans la demande internationale WO 98/48845 qui illustre notamment une émulsion huile-dans-eau dans laquelle les gouttelettes d'huile sont constituées par de l'huile de sésame renfermant un chromophore particulier, le Sudan III, qui absorbe à une longueur d'onde de 507 nm ;
- systèmes pour l'imagerie utilisant des agents de contraste à base d'émulsions. Ces systèmes ont été développés pour l'Imagerie par Résonance Magnétique (IRM) comme cela est par exemple décrit dans l'article de P.M. Winter, et al., Cancer Research, 2003, 63, 5838-5843 et pour l'imagerie par rayons X comme par exemple décrit dans la demande de brevet US 2005/0079131.

L'agent de contraste pour l'IRM conçu par Winter *et al.* (pré-cité) est une émulsion d'huile fluorocarbonée (par exemple le bromure de perfluorooctyle), dans l'eau stabilisée par une couche de tensioactifs. Le mélange de tensioactifs utilisé est notamment composé de chélates de gadolinium tels que l'acide pentaacétique de gadolinium-diéthylènetriamine-bis-oléate (Gd-DTPA-BOA) à hauteur de 30 % (mol). Ces composés sont des agents chélatants qui jouent le rôle de label IRM de par leurs propriétés magnétiques. Le ciblage de tumeurs est assuré par l'ajout d'une faible quantité de distéaroylphosphatidyléthanolamine poly(éthylène glycol-2000)-maléimide (DSPE-PEG (2000)-maléimide) couplé à un antagoniste peptidomimétique des intégrines aᵥβ₃ dans la couche de tensioactifs (0,5% mol). Les nanoparticules vont alors cibler les cellules exprimant les intégrines aᵥβ_{3,} protéines associées à l'angiogénèse accompagnant la croissance tumorale.

Les propriétés d'agents de contraste dans ces nanoparticules sont donc conférées par les tensioactifs. De plus, le diamètre de ces particules, environ 270 nm, est relativement grand et la polydispersité est importante. Les auteurs Winter *et al.* estiment d'ailleurs que la diffusion des nanoparticules vers les tumeurs est limitée par leur grande taille. Ces nanoparticules permettent cependant d'augmenter le signal d'IRM de 126 % dans les zones ciblées.

Les nanoparticules décrites dans la demande de brevet US 2005/0079131 sont également des émulsions d'huile dans l'eau, formées par un composé lipophile couplé à un élément ayant un numéro atomique (Z) élevé, tel que Z>36, stabilisées par une couche de tensioactifs à base de lécithine et de cholestérol. Dans cette formulation, préparée par microfluidiseur, c'est l'huile qui joue le rôle d'agent de contraste pour les rayons X grâce à son atome lourd Z>36. Le ciblage est là encore assuré par l'utilisation de DSPE-PEG (2000)-maléimide couplé à un antagoniste peptidomimétique des intégrines aᵥβ₃. La taille nominale des particules est inférieure à 200 nm. Cependant cette demande de brevet ne mentionne aucun test biologique *in vivo* dans l'animal vivant avec ce type d'émulsion. D'ailleurs aucun agent de furtivité n'est greffé en surface pour permettre une biodistribution homogène de l'agent de contraste dans le corps. Il est par ailleurs indiqué dans cette demande de brevet que la nanoparticule peut de plus inclure un fluorophore, à sa surface (au sein la couche de tensioactifs), celui-ci pouvant notamment être la fluorescéine, en tant qu'agent d'imagerie auxiliaire ("ancillary agent"). Il est cependant à noter que la fluorescéine n'est pas un fluorophore absorbant/émettant dans le proche infrarouge et n'est donc pas utilisable pour l'imagerie *in vivo* non invasive du petit animal. De plus, la présence d'un élément de numéro atomique élevé dans l'huile présente l'inconvénient d'inhiber la fluorescence des nanoparticules.

C'est donc afin de remédier à l'ensemble des problèmes rencontrés lors de la fabrication ou de la mise en oeuvre des systèmes existants que les Inventeurs ont mis au point ce qui fait l'objet de l'invention.

Les Inventeurs se sont en effet fixés pour but de pourvoir à une sonde fluorescente n'ayant pas les inconvénients des sondes fluorescentes actuellement disponibles et qui soit simple à préparer et à utiliser, en particulier pour l'imagerie *in vivo* non invasive du petit animal. Les inventeurs se sont plus particulièrement fixés pour objectif de pourvoir à une formulation permettant, d'une part, d'augmenter éventuellement le rendement quantique de fluorescence de composés fluorophores émettant et absorbant dans le proche infrarouge et, d'autre part, d'augmenter la durée de vie de fluorescence à une valeur supérieur à 0,5 ns, de façon à pouvoir utiliser les techniques d'imagerie de fluorescence résolue dans le temps en milieu diffusant (imagerie pulsée *in vivo*).

La présente invention a donc pour objet une émulsion fluorescente de type huile-dans-eau comprenant au moins une phase continue aqueuse et au moins un phase dispersée huileuse, ladite émulsion étant caractérisée par le fait que la phase dispersée est constituée de gouttelettes d'au moins une huile biocompatible de diamètre moyen supérieur ou égal à 10 nm et inférieur ou égal à 200 nm, que lesdites gouttelettes d'huile renferment au moins un fluorophore lipophile absorbant et émettant à une longueur d'onde comprise entre 640 et 900 nm, lesdites gouttelettes étant stabilisées par une couche tensioactive située à la périphérie desdites gouttelettes, ladite couche comprenant, en mélange, au moins un tensioactif amphiphile et au moins un co-tensioactif de furtivité.

L'huile biocompatible de l'invention est composée d'au moins une huile végétale ou animale et d'au moins une huile cristallisable riche en glycérides d'acides gras saturés en C8-C18 ayant la composition suivante:
C₈ : 0,1 à 0,9 %,
C₁₀ : 0,1 à 0,9 %,
C₁₂ : 25 à 50 %,
C₁₄ : 10 à 24,9 %,
C₁₆ : 10 à 24,9 %,
C₁₈ : 10 à 24,9 %.

Cette émulsion présente les avantages suivants :
- elle est composée de matériaux organiques biocompatibles dont une grande partie est assimilable par l'organisme dans lequel elle est susceptible d'être injectée ;
- elle est particulièrement stable au cours du temps, notamment en terme de taille des particules et de propriétés de fluorescence ;
- elle constitue une sonde moléculaire pour l'imagerie de fluorescence du petit animal *in vivo* dans laquelle les molécules organiques fluorescentes sont encapsulées et donc indétectables par l'organisme ;
- cette sonde moléculaire est conçue de manière à avoir un temps de circulation dans l'organisme important. Cela entraîne une augmentation des chances de reconnaissance cible - ligand ;
- du fait de leur présence directement au coeur des gouttelettes d'huile, c'est-à-dire à l'intérieur des gouttelettes d'huile et non à leur périphérie, un grand nombre de molécules fluorescentes est associé à la reconnaissance d'une cible par un ligand, ce qui permet une meilleure sensibilité de la détection.

Au sens de la présente Invention, le terme "gouttelette" englobe à la fois les gouttelettes d'huile liquide proprement dites ainsi que les particules solides issues d'émulsions de type huile-dans-eau dans lesquelles l'huile utilisée est une huile cristallisable. Dans ce dernier cas, on parle alors d'émulsion solide.

Le fait que le fluorophore lipophile soit incorporé directement à l'intérieur des gouttelettes d'huile apporte les avantages supplémentaires suivants :
- de protéger le fluorophore de l'environnement aqueux, dans lequel son photo-blanchiment (perte de fluorescence en présence de lumière) est plus rapide ;
- d'encapsuler un grand nombre de fluorophores tout en laissant libre les fonctions de greffage de surface des nanoparticules pour leur fonctionnalisation par les ligands biologiques. Ainsi aucune liaison de greffage en surface de la nanoparticule n'a besoin d'être sacrifiée pour le greffage de fluorophores, elles peuvent toutes être utilisées pour le greffage de ligands de ciblage, dont la densité surfacique peut ainsi être plus élevée ;
- par le choix des fluorophores présents dans le coeur des gouttelettes d'huile de l'émulsion conforme à l'invention. Les fluorophores utilisés dans l'invention sont des fluorophores lipophiles et absorbant/émettant dans le proche infrarouge ;
- par la présence d'agents de furtivité pour donner à la nanoparticule la pharmaco-dynamique adéquate : ces agents de furtivité permettent à la fois d'augmenter la stabilité des émulsions mais aussi de "leurrer" les défenses immunitaires de l'organisme ;
- par l'augmentation de la durée de vie de fluorescence et, pour certains, du rendement quantique des composés fluorophores présents au coeur des gouttelettes d'huile, ce qui permet d'accéder à l'imagerie de fluorescence résolue dans le temps (fluorescence puisée) en milieu diffusant. Il devient en particulier ainsi possible d'obtenir des rendements quantiques dans l'eau et dans les tampons aqueux inégalés pour ce domaine de longueur d'onde : de 24 % pour le iodure de 1,1'-dioctadécyl-3,3,3',3'-tétraméthylindotricarbocyanine (DiR) et de 37 % pour le perchlorate de 1,1'-dioctadécyl-3,3,3',3'-tétraméthylindodicarbocyanine (DiD) lorsque qu'ils sont formulés dans une nanoémulsion conformément à la présente invention alors qu'à notre connaissance, les rendements quantiques de fluorescence des fluorophores organiques absorbant et émettant entre 640 et 900 nm, dont les fluorophores organiques commerciaux tels que par exemple l'Alexa 750 (commercialisé par la société Invitrogen), le Cy7, le Cy7.5 (commercialisés par la société GE-Heathcare), l'IR-Dye 800 (commercialisé par la société Li-Cor) sont de 10-15 % au maximum lorsque ces fluorophores sont formulés dans l'eau ou dans un tampon aqueux.

La taille des gouttelettes d'huile au sein de l'émulsion est de préférence comprise entre 10 et 80 nm inclusivement. Les Inventeurs ont en effet observé que le seuil d'internalisation cellulaire se situe approximativement autour de 80 nm. Pour des tailles plus importantes on n'observe pas d'internalisation. L'huile biocompatible de l'invention est composée d'au moins une huile végétale ou animale et d'au moins une huile cristallisable riche en glycérides d'acides gras saturés en C8-C18 ayant la composition suivante: C8 : 0,1 à 0,9 %, C10 : 0,1 à 0,9 %, C12: 25 à 50 %, C14: 10 à 24,9 %, C16: 10 à 24,9 %, C18: 10 à 24,9 %.

Selon l'invention, les huiles biocompatibles sont choisies parmi les huiles naturelles d'origine végétale ou animale, les huiles synthétiques et leurs mélanges. Ces huiles sont utilisées sans modification chimique ou physique préalablement à la formation de l'émulsion.

Parmi de telles huiles, on peut notamment citer les huiles d'origine végétale parmi lesquelles figurent notamment les huiles de soja, de palme, d'arachide, d'olives, de lin, de pépins de raisins et de tournesol ; les huiles d'origine animale parmi lesquelles figurent notamment les huiles de poissons ; les huiles synthétiques parmi lesquelles figurent notamment les triglycérides, diglycérides et monoglycérides ; lesdites huiles pouvant être utilisées seules ou en mélanges.

Ces huiles peuvent être de première expression, raffinées ou inter-estérifiées.

L'invention est plus particulièrement basée sur des huiles choisies parmi les huiles peu solubles dans l'eau, c'est-à-dire présentant une balance hydrophile-lipophile (HLB) généralement inférieure à 8 et encore plus préférentiellement comprise entre 3 et 6 telles que par exemple l'huile de soja ou bien encore parmi les huiles cristallisables (cires) riches en glycérides d'acides gras saturés en C₈-C₁₈, principalement en C₁₂-C₁₄ tels que les mélanges de glycérides hemi synthétiques vendus sous la dénomination commerciale Suppocire® N par la société Gattefossé. Parmi de tels mélanges, le produit vendu sous la dénomination Suppocire ® NC par la société Gattefossé est particulièrement préféré ; ce produit est solide à température ambiante et est obtenu par estérification directe d'acides gras et de glycérol. Il a la composition quali-quantitative suivante :
C₈ : 0,1 à 0,9 %
C₁₀: 0,1 à 0,9 %
C₁₂: 25 à 50 %
C₁₄: 10 à 24,9 %
C₁₆: 10 à 24,9 %
C₁₈: 10 à 24,9 %.

Selon l'invention, la phase huileuse est composée d'au moins une huile végétale ou animale et d'au moins une huile cristallisable riche en glycérides d'acides gras saturés en C₈-C₁₈ ayant la composition mentionnée ci-dessus.

L'utilisation d'un tel mélange permet d'améliorer la solubilisation du ou des fluorophores au sein de la phase huileuse et ainsi d'améliorer les propriétés optiques des fluorophores encapsulés.

Dans ce cas, le rapport pondéral huile végétale ou animale / huile cristallisable riche en glycérides varie de préférence entre 10/90 et 90/10 inclusivement et encore plus préférentiellement entre 20/80 et 80/20 inclusivement.

Selon un mode préférentiel, la phase huileuse est constituée au moins à 10 % en poids d'une huile dont la viscosité est supérieure ou égale à 100 cP à 20°C (valeurs de viscosité tabulées par exemple dans le Handbook of Chemistry and Physics, CRC Press, 88th Edition, 2007). La présence dans la phase huileuse d'une telle huile permet de conférer aux fluorophores formulés dans les nanoémulsions, des durées de vie de fluorescence particulièrement adaptées à l'imagerie *in vivo* de fluorescence résolue dans le temps.

Selon l'Invention, la nature du ou des tensioactifs amphiphiles assurant la stabilisation des gouttelettes d'huile au sein de l'émulsion n'est pas critique. Ces tensioactifs amphiphiles (comportant une partie hydrophile et une partie lipophile) sont généralement choisis parmi les composés dont la partie lipophile comprend une chaîne saturée ou insaturée, linéaire ou ramifiée, ayant de 8 à 30 atomes de carbone. Ils peuvent être choisis parmi les phospholipides, les cholestérols, les lysolipides, les sphingomyélines, les tocophérols, les glucolipides, stéarylamines, les cardiolipines d'origine naturelle ou synthétique ; les molécules composées d'un acide gras couplé à un groupement hydrophile par une fonction éther ou ester tels que les esters de sorbitan comme par exemple les monooléate et monolaurate de sorbitan vendus sous les dénominations Span® par la société Sigma ; les lipides polymérisés ; les lipides conjugués à de courtes chaînes d'oxyde de polyéthylène (PEG) tels que les tensioactifs nonioniques vendus sous les dénominations commerciales Tween® par la société ICI Americas Inc. et Triton® par la société Union Carbide Corp. ; les esters de sucre tels que les mono- et di-laurate, mono- et di-palmitate, mono- et distéarate de saccharose ; lesdits tensioactifs pouvant être utilisés seuls ou en mélanges.

Selon l'invention, le ou les tensioactifs sont de préférence des tensioactifs d'origine naturelle et assimilables (biocompatibles) comme la lécithine de soja, les phospholipides et le cholestérol.

La nature du ou des fluorophores lipophiles utilisables dans l'émulsion conforme à la présente invention n'est pas critique à partir du moment où ils sont compatibles avec l'imagerie de fluorescence *in vivo* et qu'ils absorbent et émettent à une longueur d'onde comprise entre 640 et 900 nm. En effet, pour que la lumière d'excitation et la lumière émise par le fluorophore puissent traverser les tissus, il convient d'utiliser des fluorophores absorbant et émettant dans le proche infrarouge. A titre de fluorophore lipophile on peut par exemple citer les composés décrits dans le chapitre 13 ("Probes for Lipids and Membranes") du catalogue InVitrogen.

De façon plus précise, on peut notamment citer, à titre de fluorophore, les analogues d'acides gras; les sphingolipides, les stéroïdes, les lipo-polysaccharides et les phospholipides fonctionnalisés par un groupement absorbant et émettant dans le proche infrarouge (640-900 nm) et leurs dérivés amphiphiles. Parmi de tels fluorophores on peut plus particulièrement citer les dérivés des cyanines, des rhodamines, des fluorescéines, des coumarines, des squaraines, des azulènes, des xanthènes, des oxazines et des 4,4-difluoro-4-bora-3a,4a-diaza-s-indacène ("boron dipyrromethene" en anglais), ainsi que dérivés amphiphiles desdits fluorophores.

A titre d'exemple, on peut plus particulièrement mentionner les produits fluorescents vendus sous les dénominations commerciales Bodipy ® 665/676 (Ex/Em.) par la société Invitrogen; les dérivés amphiphiles de dialkylcarbocyanines telles que le perchlorate de 1,1'-dioctadécyl-3,3,3',3'-tétraméthylindodicarbocyanine (DiD) vendu sous la référence D-307 par la société Invitrogen et l'iodure de 1,1-dioctadécyl-3,3,3',3'-tétraméthylindotricarbocyanine (DiR) vendu sous la référence D-12731 par la société Invitrogen.

Selon une forme de réalisation préférée de l'Invention, les fluorophores sont choisis parmi les dérivés amphiphiles de dialkylcarbocyanines.

Le ou les co-tensioactifs de furtivité utilisables dans les émulsions conformes à la présente Invention sont de préférence des molécules amphiphiles dont la partie hydrophile est totalement ou en partie composée d'une chaîne d'oxyde de polyéthylène (PEO ou PEG) et dans laquelle le nombre de motifs PEO varie de préférence entre 2 et 500. Les co-tensioactifs de furtivité peuvent aussi être composés de poly-saccharides de type dextrans par exemple. A titre d'exemple de co-tensioactifs de furtivité utilisables selon la présente Invention, on peut en particulier citer les composés conjugués polyéthylèneglycol/phosphatidyl-éthanolamine (PEG-PE), les éthers d'acide gras et de polyéthylèneglycol tels que les produits vendus sous les dénominations commerciales Brij ® (par exemple Brij ® 35, 58, 78 ou 98) par la société ICI Americas Inc., les esters d'acide gras et de polyéthylèneglycol tels que les produits vendus sous les dénominations commerciales Myrj ® par la société ICI Americas Inc. (par exemple Myrj ® 45, 52, 53 ou 59) et les copolymères blocs d'oxyde d'éthylène et d'oxyde de propylène tels que les produits vendus sous les dénominations commerciales Pluronic ® par la société BASF AG (par exemple Pluronic ® F68, F127, L64 ou L61) ou les produits vendus sous la dénomination commerciale Synperonic ® par la société Unichema Chemie BV (par exemple Synperonic ® PE/F68, PE/L61 ou PE/L64).

Selon une forme de réalisation préférée de l'invention, la couche tensioactive située à la périphérie des gouttelettes d'huile de l'émulsion comprend en outre au moins un agent de ciblage d'une activité biologique d'intérêt, ledit agent de ciblage étant constitué d'un co-tensioactif de greffage amphiphile dont la partie hydrophile est liée, de façon covalente, à un ligand biologique. La présence d'un agent de ciblage permet de cibler un processus biologique d'intérêt particulier.

Selon une forme de réalisation avantageuse de l'Invention, lesdits agents de ciblage sont choisis parmi les composés de formule (I) suivante : 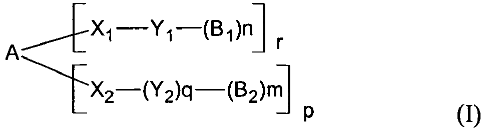 dans laquelle :
- A est la partie lipophile d'un co-tensioactif de greffage amphiphile (CoTA),
- X₁ et X₂, identiques ou différents, constituent la partie hydrophile dudit co-tensioactif CoTA et sont composés d'un bras espaceur flexible choisi parmi les chaînes carbonées linéaires ou ramifiées, saturées ou insaturées, éventuellement substituées, interrompues et/ou terminées par un ou plusieurs hétéroatomes choisis par exemple parmi N, O, P et S, et/ou par un ou plusieurs groupements choisis par exemple parmi les radicaux alkyle en C₁-C₄, alcoxy en C₁-C₄, aryle ou par une ou plusieurs fonctions choisies parmi les fonctions éther, ester, amide, carbonyle, carbamate, urée, thiourée et disulfure ;
- Y₁ et Y₂, identiques ou différents, sont choisis parmi les groupements chimiques aptes à relier X₁ et B₁, respectivement X₂ et B₂, par des liaisons covalentes ;
- B₁ et B₂, identiques ou différents, sont des ligands biologiques dont l'une des extrémités est engagée dans la liaison covalente formée avec X₁, respectivement X₂ ;
- n est un nombre entier compris entre 1 et 20 inclusivement ;
- q est un nombre entier égal à 0 ou 1 ;
- m est un nombre entier compris entre 0 et 20 inclusivement, étant entendu que m = 0 lorsque q = 0 ;
- p est un nombre entier compris entre 0 et 10 inclusivement ; et
- R est un nombre entier compris entre 0 et 10 inclusivement.

La partie lipophile (A) du co-tensioactif de greffage CoTA présent dans l'agent de ciblage de formule (I) lui permet de s'ancrer à la surface des gouttelettes d'huile au sein de la couche tensioactive périphérique. Elle peut notamment être composée d'une chaîne alkyle en C₆-C₂₆, linéaire ou ramifiée, saturée ou insaturée.

La partie hydrophile du CoTA constituant les bras espaceurs X₁ et X₂ des composés de formule (I) ci-dessus peut notamment être choisie parmi les chaînes constituées de motifs polyoxyéthylène ou dextran.

Selon une forme de réalisation avantageuse de l'Invention, les liaisons covalentes (groupements fonctionnels Y₁/Y₂) assurant la fixation de X₁/X₂ aux unités B₁/B₂ sont issues de la réaction entre une fonction chimique initialement portée par la partie hydrophile du CoTA avant sa réaction avec B₁/B₂ et une fonction chimique complémentaire portée par les ligands biologiques B₁/B₂ avant leur réaction avec X₁ respectivement X₂. A titre d'exemple non limitatif et non exhaustif, on peut notamment citer les liaisons covalentes résultant de la réaction :
- d'une amine et d'un ester activé, par exemple par un groupement N-succinimidyle conduisant à la formation de liaisons amide ;
- d'une oxyamine et d'un aldéhyde conduisant à la formation de liaisons oxyme ; et
- d'un maléimide et d'un thiol conduisant à la formation de liaisons thioéther.

Parmi les ligands biologiques utilisables à titre d'unités B₁/B₂ des agents de ciblage de formule (I) ci-dessus, on peut notamment citer :
i) les ligands biologiques permettant de cibler spécifiquement certaines cellules tels que des peptides par exemple le peptide RGD (linéaire ou cyclisé), leurs dérivés et leurs analogues (ex : le peptide octéotrate, analogue de la somatostatine, un analogue de la bombésine, de la neurotensine, l'EGF, le VIP...) ; des protéines, des anti-corps, leurs dérivés ou leurs analogues ; des monosaccharides tels que le glucose, des oligosaccharides, des polysaccharides, leurs dérivés et leurs analogues ; des oligonucléotides, ADN, leurs dérivés et leurs analogues ; des molécules organiques telle que le folate, le pamidronate biphosphonaté et des complexes organométalliques dont l'activité de ciblage est due à la reconnaissance moléculaire de ces ligands par des récepteurs sur-exprimés à la surface des cellules de la zone d'intérêt ;
ii) les ligands biologiques marqueurs d'une activité biologique donnée, par exemple d'une activité enzymatique. A titre d'exemple de tels ligands, on peut par exemple mentionner les peptides clivables par une protéase donnée, à l'extrémité desquels sera greffé un inhibiteur de la fluorescence du label. Ce type de ligands permet d'imager spécifiquement l'activité enzymatique de la protéase (C.H. Tung, pré-cité). Un autre exemple est constitué par les ligands biologiques comportant un pont disulfure séparant le label d'un inhibiteur de sa fluorescence. Un tel ligand biologique permet alors d'imager spécifiquement l'internalisation de la sonde dans une cellule comme décrit par exemple dans la demande de brevet FR 2 888 938.

Le couplage des ligands biologiques sur les co-tensioactifs de greffage CoTA peut être réalisé soit avant l'émulsification soit après l'émulsification. Dans ce dernier cas, il faut que les réactions chimiques employées soient compatibles avec la stabilité colloïdale des émulsions. Elles doivent notamment se dérouler en solution aqueuse et à un pH ni trop acide ni trop basique (pH 5-11).

La phase continue de l'émulsion conforme à l'Invention est une phase aqueuse, de préférence constituée d'eau et/ou d'un tampon physiologiquement acceptable tel qu'un tampon phosphate comme par exemple du PBS ("Phosphate Buffer Saline") ou d'une solution de chlorure de sodium.

L'émulsion conforme à l'Invention peut être préparée par toute méthode classique connue de l'homme de l'art pour la préparation des émulsions, par exemple selon un procédé comprenant les étapes suivantes :
a) la préparation d'un prémélange huileux pour la phase dispersée de l'émulsion consistant à mélanger les différents constituants huileux biocompatibles dans un solvant organique tel que par exemple le chloroforme pour obtenir, après évaporation du solvant, un prémélange huileux homogène pour la phase dispersée,
b) la préparation proprement dite de la phase dispersée de l'émulsion par mélange homogène dudit prémélange huileux avec au moins un fluorophore lipophile absorbant et émettant dans le proche infrarouge ;
c) la préparation de la phase continue de l'émulsion par mélange, en phase aqueuse, de préférence à chaud, d'au moins un tensioactif amphiphile, d'au moins un co-tensioactif de furtivité et éventuellement d'au moins un agent de ciblage d'une activité biologique d'intérêt, ledit agent de ciblage étant constitué d'un co-tensioactif de greffage amphiphile dont la partie hydrophile est liée, de façon covalente, à un ligand biologique ;
d) l'ajout de la phase continue à la phase dispersée et l'émulsification du mélange résultant jusqu'à l'obtention d'une émulsion homogène dans laquelle le diamètre moyen des gouttelettes d'huile est supérieur à 10 nm et inférieur à 200 nm. Cette émulsification peut par exemple être réalisée à l'aide d'un sonificateur, pendant une durée comprise entre 4 et 10 minutes.

Selon une forme de réalisation particulière, et lorsque la phase huileuse de la nanoémulsion est composée d'au moins une huile végétale ou animale et d'au moins une huile cristallisable riche en glycérides d'acide gras en C₈-C₁₈, le tensioactif utilisé pour stabiliser la nanoémulsion peut être incorporé en totalité ou en partie à la phase dispersée lors de l'étape b) ci-dessus. Cette forme de réalisation permet d'éviter la formation de liposomes lors de la préparation de la nanoémulsion conforme à l'invention et est particulièrement avantageuse lorsque ledit tensioactif est de la lécithine de soja.

Avant son utilisation, l'émulsion est ensuite de préférence diluée, par exemple au demi et stérilisée par exemple par filtration. Cette étape de filtration permet par ailleurs d'éliminer les éventuels agrégats qui pourraient s'être formés au cours de la préparation de l'émulsion.

Ainsi que cela a été amplement décrit et explicité ci-avant, les émulsions fluorescentes conformes à l'Invention peuvent être utilisées pour la détection d'une activité biologique d'intérêt *in vivo.*

La présente Invention a donc pour deuxième objet, le réactif de diagnostic d'une activité biologique d'intérêt, caractérisé par le fait qu'il comprend au moins une émulsion fluorescente conforme à l'Invention et telle que décrite ci-dessus.

Selon une forme de réalisation particulière et préférée de l'invention, le réactif est un réactif de diagnostic *in vivo.*

Enfin, l'Invention a pour objet, l'utilisation d'au moins une émulsion fluorescente telle que décrite précédemment pour la préparation d'un réactif de diagnostic d'une activité biologique d'intérêt *in vivo* par imagerie de fluorescence, et en particulier par imagerie de fluorescence résolue dans le temps (fluorescence puisée) et/ou pour l'aide au développement et à l'optimisation d'outils thérapeutiques, comme des médicaments. En effet, un tel réactif peut permettre :
- la détection de cellules cancéreuses chez l'animal, éventuellement chez l'homme, par imagerie de fluorescence, préférentiellement par imagerie de fluorescence non invasive ;
- la détection de plaques d'athéromes chez l'animal, éventuellement chez l'homme, par imagerie de fluorescence, préférentiellement par imagerie de fluorescence non invasive.
- la détection de fibres β-amyloïdes caractéristiques des maladies neuro-dégénératives chez l'animal, éventuellement chez l'homme, par imagerie de fluorescence, préférentiellement par imagerie de fluorescence non invasive.
- le suivi *in vivo* de processus enzymatiques chez l'animal, éventuellement chez l'homme, par imagerie de fluorescence, préférentiellement par imagerie de fluorescence non invasive ;
- le suivi *in vivo* de l'expression de gènes chez l'animal, par imagerie de fluorescence, préférentiellement par imagerie de fluorescence non invasive.
- l'évaluation d'une thérapie chez l'animal, par imagerie de fluorescence, préférentiellement par imagerie de fluorescence non invasive ; ou bien encore
- le suivi de la biodistribution d'une drogue, de sa délivrance contrôlée, de son efficacité.

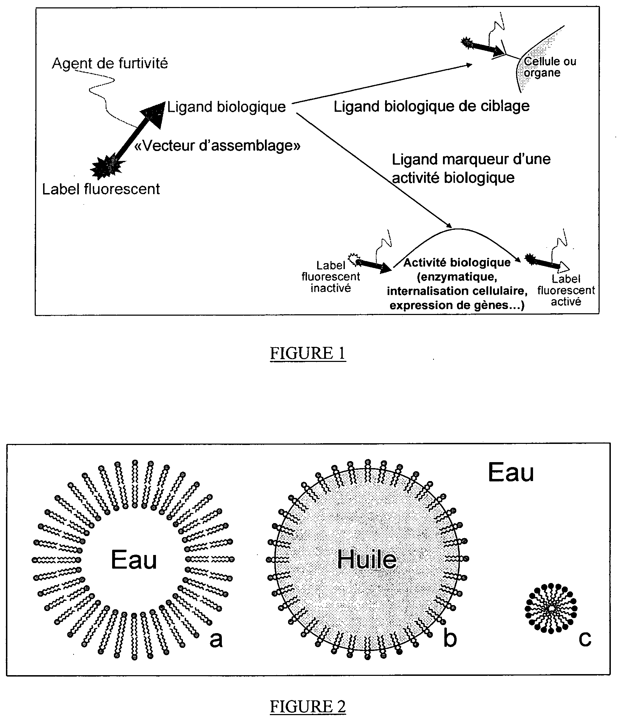

Outre les dispositions qui précèdent, l'Invention comprend encore d'autres dispositions qui ressortiront de la description qui va suivre, qui se réfère à des exemples de préparation d'émulsions fluorescentes conformes à la présente invention, à un exemple comparatif mettant en oeuvre une telle émulsion pour l'imagerie par fluorescence *in vivo,* et à des exemples démontrant l'effet de la formulation de composés fluorophores sous la forme de nanoémulsion sur la durée de vie de fluorescence et le rendement quantique de fluorescence, ainsi qu'aux figures 1 à 11 annexées dans lesquelles :
- la figure 1 est une représentation schématique des différentes entités pouvant constituer une sonde pour l'imagerie de fluorescence ;
- la figure 2 est une représentation comparative de la structure d'une nano-émulsion (b), par rapport aux systèmes micellaires (c) et aux polymersomes (a), les structures (a), (b) et (c) ayant toute pour point commun d'avoir de l'eau pour phase continue de la dispersion ;

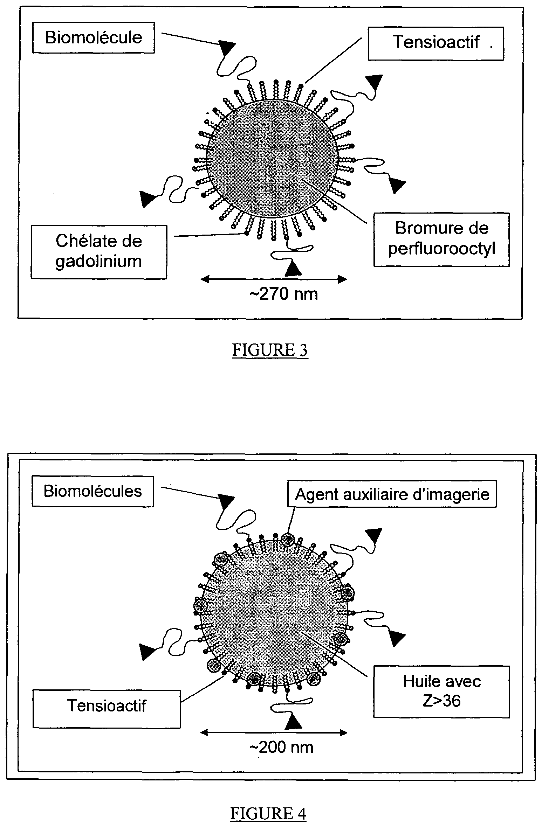

- la figure 3 correspond au schéma de principe de l'agent de contraste décrit par Winter *et al.,* (pré-cité), constitué d'une émulsion huile-dans-eau dans laquelle la phase continue aqueuse renferme une dispersion de gouttelettes (diamètre environ 270 nm) de bromure de perfluorooctyle stabilisées par des tensioactifs notamment composés de chélates de gadolinium sur certains desquels sont fixés du DSPE-PEG (2000)-maléimide couplé à un antagoniste peptidomimétique des intégrines aᵥβ₃ ;
- la figure 4 correspond au schéma de principe de l'agent de contraste décrit dans la demande de brevet US 2005/0079131, celui-ci se présentant sous la forme d'une émulsion huile-dans-eau dans laquelle la phase continue aqueuse renferme une dispersion de gouttelettes d'huile (diamètre moyen d'environ 200 nm) incorporant un composé lipophile couplé à un élément de numéro atomique > à 36, lesdites gouttelettes étant stabilisées par une couche de tensioactifs dans laquelle s'insère un agent auxiliaire d'imagerie, le ciblage des biomolécules étant assuré par le DSPE-PEG (2000)-maléimide couplé à un antagoniste peptidomimétique des intégrines aᵥβ₃;

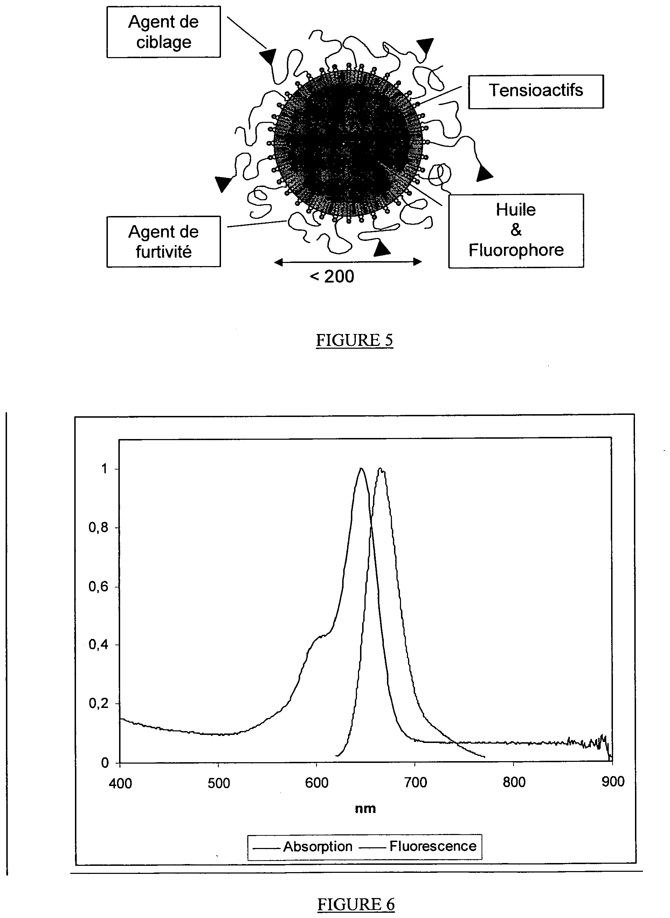

- la figure 5 correspond au schéma de principe de l'émulsion huile-dans-eau conforme à la présente invention, dans laquelle la phase continue aqueuse renferme une dispersion de gouttelettes d'huile (diamètre moyen < 200 nm) au coeur desquelles est encapsulé au moins un fluorophore lipophile, lesdites gouttelettes étant stabilisée par une couche périphérique tensioactive comprenant au moins un tensioactif, au moins un co-tensioactif de furtivité et au moins un agent de ciblage d'une activité biologique d'intérêt ;
- la figure 6 représente le spectre d'absorption/émission d'une émulsion conforme à la présente invention dans laquelle les gouttelettes d'huile sont constituées d'huile de soja renfermant le fluorophore DiD vendu sous la référence D-307 par la société Invitrogen. Sur cette figure, l'intensité exprimée en unités arbitraire est fonction de la longueur d'onde exprimée en nm, l'absorption correspondant à la courbe de gauche et l'émission de la fluorescence à la courbe légèrement décalée vers la droite ;

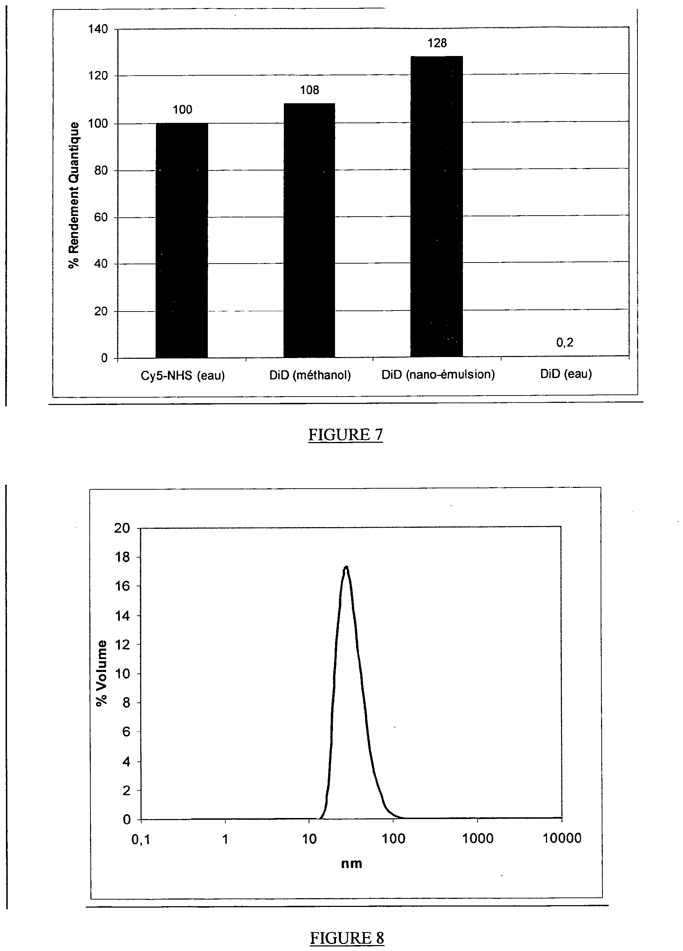

- la figure 7 montre les rendements quantiques du fluorophore DiD en fonction de son milieu (en solution dans le méthanol, incorporé dans les nano-émulsions et en solution dans l'eau) par rapport à celui du Cy5-NHS (Amersham) dont la valeur a été arbitrairement fixée à 100 ;
- la figure 8 représente la distribution en taille pondérée en volume d'une émulsion de la présente invention préparée suivant l'exemple 1. Sur cette figure, le pourcentage en volume est fonction de la taille en nm ;

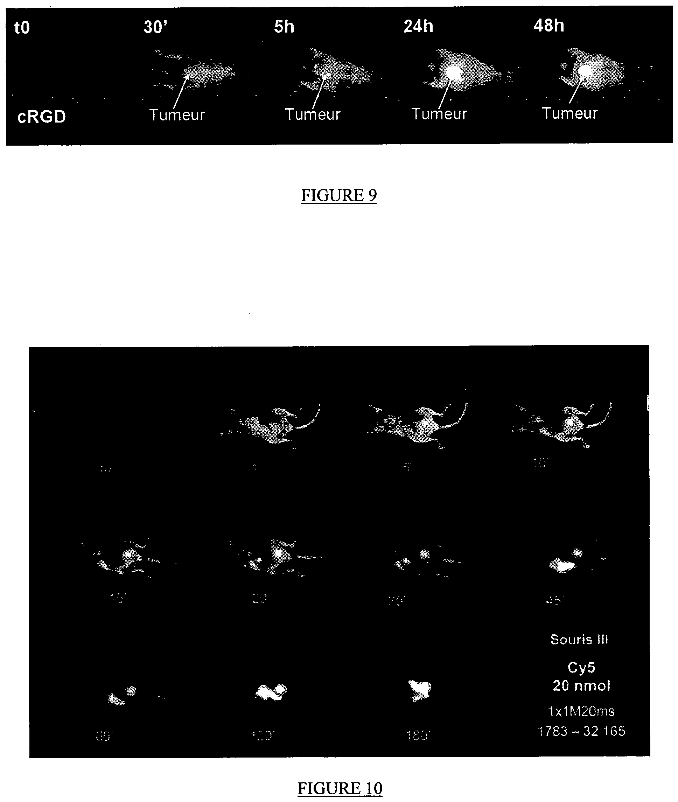

- la figure 9 montre l'évolution de la distribution au cours du temps chez une souris porteuse d'une tumeur Ts/Apc (carninome du sein, modèle murin), d'une émulsion fluorescente conforme à la présente invention, c'est-à-dire contenant le fluorophore DiD et fonctionnalisée par des cRGD tel que décrit dans l'exemple 3. La distribution montre une accumulation importante dans la zone tumorale ;
- la figure 10 montre l'évolution de la distribution, au cours du temps chez la souris, d'une sonde fluorescente ne faisant pas partie de l'invention, c'est-à-dire une cyanine Cy5-NHS (Amersham), qui est un analogue hydrophile du fluorophore DiD. La cyanine n'est pas encapsulée dans une émulsion et ne possède pas d'agent de ciblage. Le fluorophore est d'abord stocké dans les reins puis s'élimine ;

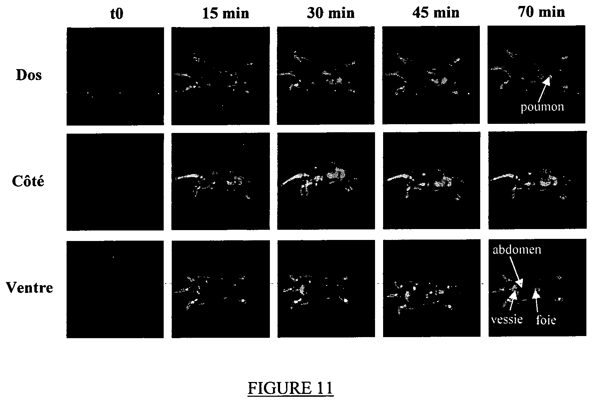

- la figure 11 montre l'évolution de la distribution au cours du temps chez la souris, d'une émulsion fluorescente conforme à la présente invention, c'est-à-dire contenant le fluorophore DiD, sans agent de ciblage. La distribution des nanoparticules dans l'organisme est très homogène et il n'y a pas d'élimination.

Il doit être entendu toutefois que ces exemples ne sont donnés qu'à titre purement illustratif de l'invention.

### EXEMPLE 1: PREPARATION DE NANO-EMULSIONS FLUORESCENTES NON FONCTIONNALISEES

### 1) Préparation d'un pré-mélange pour la phase dispersée

On a préparé un pré-mélange constitué de 15 % p/p d'huile de soja (Sigma-Aldrich), de 45 % p/p de glycérides semisynthétiques vendus sous la dénomination commerciale Suppocire® NC (Gattefossé) et de 40 % p/p de lécithine de soja (enrichie à 75 % de phosphatidylcholine) vendue par la société Lipoïd sous la dénomination commerciale Lipoïd® S75. Ces composés ont été dissous dans du chloroforme, la solution a ensuite été évaporée sous pression réduite et séchée à 50°C de manière à obtenir un pré-mélange se présentant sous la forme d'une huile visqueuse qui se solidifie en refroidissant.

### 2) Préparation de la phase dispersée

On a prélevé, à une température d'environ 70°C, 0,833 g du pré-mélange préparé ci-dessus à l'étape précédente, auquel on a ajouté 1,5 mg de perchlorate de 1,1'-dioctadécyl-3,3,3',3'-tétraméthylindodicarbocyanine (DiD) qui est un fluorophore huileux vendu sous la référence D-307 par la société Invitrogen. La solution a été homogénéisée par vortex, et maintenue à 70°C en prévision de la phase d'émulsification.

### 3) Préparation de la phase continue

La phase continue était composée de 0,125 g de glycérol, de 0,55 g de stéarate de polyoxyéthylène à 50 moles d'oxyde d'éthylène vendu sous la dénomination commerciale Myrj® 53 par la société ICI Americas Inc. et d'une solution tampon phosphate (Phosphate Buffer Solution : PBS) pour compléter à 4,166 g. Cette solution a été chauffée à 70°C en prévision de l'émulsification.

### 4) Emulsification

La phase continue a été ajoutée à la phase dispersée et le mélange a été émulsionné pendant 6 minutes pour une énergie totale de 6 000 Joules avec un sonificateur AV505 ® équipé d'une sonde conique de 3 mm de diamètre (Sonics, Newtown).

### 5) Préparation pour l'injection intraveineuse chez la souris

L'émulsion précédemment obtenue a alors été diluée par un facteur 6,25 avec du PBS, puis filtrée sur un filtre de 0,22 µm en fluorure de polyvinylidène (PVDF) vendu sous la dénomination commerciale Millex®-GV par la société Millipore Corporation de façon à éliminer les agrégats, mais aussi de la stériliser.

Cette émulsion peut ensuite être directement utilisée comme sonde fluorescente pour l'imagerie fonctionnelle *in vivo.*

Les spectres d'absorption et d'émission de cette émulsion sont représentés sur la figure 6 annexée sur laquelle l'intensité exprimée en unités arbitraire est fonction de la longueur d'onde exprimée en nm ; sur cette figure l'absorption correspond à la courbe de gauche et l'émission de la fluorescence à la courbe légèrement décalée vers la droite.

Le rendement quantique de cette émulsion est 1,3 fois supérieur à celui du Cy5-NHS, comme le montre la figure 7 annexée.

La taille nominale des nanoparticules, déterminée par diffusion dynamique de la lumière sur un dispositif vendu sous la référence ALV-5000/EPP par la société ALV, était inférieure à 70 nm. La courbe de distribution des tailles est représentée sur la figure 8 annexée.

### EXEMPLE 2 : PREPARATION DE NANO-EMULSIONS FLUORESCENTES FONCTIONNALISEES AVANT EMULSIFICATION

### 1) Préparation d'un peptide de ciblage fonctionnalisé par un co-tensioactif de greffage

Un peptide cyclique de ciblage des intégrines αᵥβ₃ surexprimées à la surface des cellules endothéliales, c(RGDf[ε-S-acétylthioacétyl])K (identifié SEQ ID NO:1 dans la liste de séquence annexée) vendu par la société Ansynth Service BV (Pays-Bas) et dénommé cRGD dans ce qui suit possédant un groupement thiol protégé sous la forme d'un acide mercaptoacétique, a été couplé à un co-tensioactif de greffage le distéaroylphosphatidyléthanolamine poly(éthylène glycol-2000)-maléimide, (à savoir le DSPE-PEG(2000)-maléimide vendu par la société Avanti Polar Lipids, Inc.). Celui-ci a été mélangé au cRGD avec un ratio molaire 1:1 dans une solution tampon acide sulfonique de (4-(2-hydro xyéthyl)-1-pipérazineéthane/acide éthylène-diamine-tétraacétique (HEPES/EDTA) avec une concentration en hydroxylamine de 0,05 M. La solution a été agitée lentement sous un léger flux d'argon à température ambiante pendant 4 heures, évaporée sous pression réduite, puis redissoute dans du chloroforme en vue de la deuxième étape.

### 2) Préparation d'un pré-mélange pour la phase dispersée

Le pré-mélange était constitué de 15 % p/p d'huile de soja (Sigma-Aldrich), de 45 % p/p de Suppocire® NC (Gattefossé), de 38 % p/p de Lécithine® S-75 (Lipoïd) et de 2 % p/p de DSPE-PEG(2000)-maléimide (Aventi Polar Lipids, Inc.) couplé au cRGD. Ces composés ont été dissous dans du chloroforme, la solution a ensuite été évaporée sous pression réduite et séchée à 50°C de manière à obtenir une huile visqueuse qui se solidifie en refroidissant.

### 3) Préparation de la phase dispersée

On a prélevé, à une température d'environ 70°C, 0,833 g du pré-mélange préparé ci-dessus à la deuxième étape auquel on a ensuite ajouté 1,5 mg du fluorophore D-307 (Invitrogen). La solution a ensuite été homogénéisée par vortex, et maintenue à 70°C en prévision de la phase d'émulsification.

### 4) Préparation de la phase continue

La phase continue était composée de 0,125 g de glycérol, de 0,55 g de Myrj® 53 et d'une solution tampon PBS pour compléter à 4,166 g. Cette solution a été chauffée à 70°C en prévision de l'émulsification.

### 5) Emulsification

La phase continue a été ajoutée à la phase dispersée et le mélange a ensuite été émulsionné pendant 6 minutes pour une énergie totale de 6 000 Joules avec un sonificateur AV505 ® équipé d'une sonde conique de 3 mm de diamètre (Sonics, Newtown).

### 6) Préparation pour l'injection intraveineuse chez la souris

L'émulsion précédemment obtenue a ensuite été diluée par un facteur 6,25 avec du PBS, puis filtrée sur filtre de 0,22 µm de façon à éliminer les agrégats, mais aussi de la stériliser.

Cette émulsion peut ensuite être directement utilisée comme sonde fluorescente pour l'imagerie fonctionnelle *in vivo.*

### EXEMPLE 3 : PREPARATION DE NANO-EMULSIONS FLUORESCENTES FONCTIONNALISEES APRES EMULSIFICATION

### 1) Préparation d'un pré-mélange pour la phase dispersée

Le pré-mélange était constitué de 15 % p/p d'huile de soja (Sigma-Aldrich), de 45 % p/p de Suppocire ® NC (Gattefossé), de 38 % p/p de lécithine de soja S-75 (Lipoïd) et de 2 % p/p de DSPE-PEG(2000)-maléimide (Aventi Polar Lipids). Ces composés ont été dissous dans du chloroforme, puis la solution a ensuite été évaporée sous pression réduite et séchée à 50°C de manière à obtenir une huile visqueuse qui se solidifie en refroidissant.

### 2) Préparation de la phase dispersée

On a prélevé à une température d'environ 70°C, 0,833 g du pré-mélange décrit ci-dessus dans 1), auquel on a ajouté 1,5 mg du fluorophore DiD vendu sous la référence D-307 par la société Invitrogen. La solution a été homogénéisée par vortex et maintenue à 70°C en prévision de la phase d'émulsification.

### 3) Préparation de la phase continue

La phase continue était composée de 0,125 g de glycérol, de 0,55 g de Myrj® 53 et d'une solution tampon PBS pour compléter à 4,166 g. Cette solution a été chauffée à 70°C en prévision de l'émulsification.

### 4) Emulsification

La phase continue a été ajoutée à la phase dispersée et le mélange a été émulsionné pendant 6 minutes pour une énergie totale de 6 000 Joules avec un sonificateur AV505 ® équipé d'une sonde conique de 3 mm de diamètre (Sonics, Newtown).

### 5) Fonctionnalisation

L'émulsion a été diluée dans un tampon HEPES/EDTA contenant 0,05 M d'hydroxylamine. La solution a été désoxygénée pendant 30 minutes par un flux d'argon, puis 2 mg du peptide cRGD porteur d'un groupe thiol protégé (c(RGDfK(Ac-S-CH₂CO)), Ansynth service BV, Pays-Bas) ont été ajoutés. Le mélange réactionnel a été agité lentement sous un léger flux d'argon à température ambiante pendant 4 heures. La solution a ensuite été dialysée contre du PBS avec une membrane de dialyse Spectra/Por ® ayant un seuil de coupure égal à 12000 de manière à éliminer les cRGDs n'ayant pas réagi.

### 6) Préparation pour l'injection intra-veineuse chez la souris

L'émulsion précédemment obtenue a été diluée par un facteur 6,25 avec du PBS, puis filtrée sur un filtre de 0,22 µm de façon à éliminer les agrégats, mais aussi de la stériliser.

Cette émulsion peut ensuite être directement utilisée comme sonde fluorescente pour l'imagerie fonctionnelle *in vivo,* et notamment pour la détection de tumeur chez la souris.

A cet effet, des souris Nude femelles de 5-6 semaines (IFFA-Credo, Marcy l'Etoile, France), et maintenues sous conditions sans pathogènes ont utilisées comme modèle animal. Les cellules Ts/Apc (modèle de cancer du sein murin) ont été cultivées dans un milieu de culture RPMI 1640 comportant 10 % de sérum de veau foetal, 50 U/mL de pénicilline, 50 µg/mL de streptomycine, 50 µg/mL de 2-mercaptoéthanol à 2.5 10⁻⁵ M (tous ces produits étant commercialisés par la société Sigma-Aldrich). Les cellules ont été maintenues à 37°C sous atmosphère humide avec 5 % de CO₂. 10⁶ cellules ont ensuite été injectées en sous-cutané dans le dos des souris 2 semaines avant injection de la nanoémulsion telle que préparée ci-dessus dans cet exemple. L'injection a été réalisée dans la queue par voie intra-veineuse à raison de 200 µL de solution par souris. Toutes les injections et acquisitions d'image ont été réalisées alors que les souris étaient maintenues sous anesthésie générale par voie gazeuse (isoflurane).

Les animaux anesthésiés ont été imagés avec un dispositif d'imagerie de fluorescence par réflectance (FRI = "*Fluorescence Reflectance Imaging*"), comportant comme source d'excitation une couronne de LEDs munies de filtres interférentiels, émettant à 633 nm (puissance d'éclairement 50 µW.cm⁻²) comme décrit par exemple dans l'article de Texier, I. et al., "Luminescent probes for optical in vivo imaging", Proceedings of the SPIE, 2005, 5704, 16-22. Les images ont été recueillies après filtration par un filtre coloré RG665 de densité optique >5 à la longueur d'onde d'excitation par une caméra CCD (Orca BTL, Hamamatsu) avec un temps d'exposition de 20 ms. Les signaux ont été quantifiés à l'aide d'un logiciel de traitement d'images. Les images enregistrées 30 minutes, 5 heures, 24 heures et 48 heures après injection de la nanoémulsion fonctionnalisée par le cRGD et encapsulant le fluorophore DiD, montrent une accumulation progressive du signal de fluorescence dans la tumeur au cours du temps (figure 9). L'image t0 de la figure 9 représente une image de fluorescence de la souris avant injection de la nanoémulsion.

### EXEMPLE 4 : BIODISTRIBUTION COMPARATIVE DE NANOPARTICULES NON FONCTIONNALISEES ET D'UN FLUOROPHORE HYDROPHILE NON ENCAPSULE APRES INJECTION INTRA-VEINEUSE CHEZ LA SOURIS

200 µL de l'émulsion préparée ci-dessus à l'exemple 1 (émulsion utilisant le fluorophore DiD commercialisé par Invitrogen sous la référence D-307), correspondant à 10 nmoles de fluorophore, ont été injectés par voie intraveineuse dans la queue de souris Nude femelles de 6 à 8 semaines, et maintenues sous conditions sans pathogènes (IFFA-Credo, Marcy l'Etoile, France). Les animaux anesthésiés ont été imagés avec le dispositif d'imagerie de fluorescence par réflectance décrit précédemment dans l'exemple 3.

A titre comparatif, 200 µl d'une composition comparative renfermant 20 nmoles d'un fluorophore Cy5-NHS (Amersham) ne se présentant pas sous la forme d'une émulsion, ont été également injectées à des souris, dans les mêmes conditions que pour l'émulsion de l'exemple 1.

Les résultats obtenus ont été reportés sur la figure 10 annexée qui montre l'évolution de la distribution au cours du temps de la cyanine Cy5-NHS (Amersham). On peut notamment y voir que les reins et la vessie accumulent rapidement le fluorophore, limitant le temps où celui-ci peut être imagé dans l'organisme.

A titre comparatif, la figure 11 annexée montre l'évolution de la distribution des gouttelettes chargées en fluorophore DiD au cours du temps. On peut notamment voir que même si certains organes captent dans un premier temps une partie importante des gouttelettes chargées en fluorophore (comme le foie ou les poumons) la fluorescence s'homogénéise avec le temps. Ces résultats montrent donc que l'encapsulation du fluorophore au sein des gouttelettes d'huile améliore le temps de circulation et réduit son élimination par certains organes comme l'intestin, le foie ou les reins, permettant une meilleure exploration de l'ensemble du corps de l'animal.

### EXEMPLE 5 : ETUDE DE L'EFFET DE L'ENCAPSULATION D'UN FLUOROPHORE DANS DES NANOPARTICULES LIPIDIQUES SUR LA DUREE DE VIE DE LA FLUORESCENCE

Cet exemple, pour but de démontrer l'effet de l'encapsulation d'un composé fluorophore dans une phase huileuse sur la durée de vie de la fluorescence.

Pour ce faire, un fluorophore lipidique commercialisé par Invitrogen, le DiR, a été encapsulé dans des nanoparticules lipidiques de type émulsions huile-dans-l'eau suivant le procédé décrit ci-dessus à l'exemple 1.

La composition de ces nanoémulsions comportant un coeur lipidique composé à 75 % en poids de Suppocire ® NC et à 25 % en poids d'huile de soja est donnée dans le Tableau 1 ci-après :

**TABLEAU 1**

| **Quantités pour 5 g de nanoémulsion** | | | |
|---|---|---|---|
| | | Masse (en mg) | % |
| **Phase dispersée** | Huile de soja | 125 | 2,5 |
| | Suppocire ® NC | 375 | 7,5 |
| **Tensioactifs** | Lécithine | 350 | 7 |
| | Myrj ® 53 | 550 | 11 |
| **Phase aqueuse** | Glycérol | 125 | 2,5 |
| | PBS | 3475 | 69,5 |

200 µL du fluorophore DiR en solution dans le DMSO à une concentration de 10 mM et la lécithine ont été ajoutés au mélange d'huile (huile de soja / Suppocire ® NC). Le mélange obtenu a été chauffé à 50-60°C. Le glycérol et le tensioactif (Myrj ® 53) ont été dispersés dans la solution de PBS. La solution a été maintenue à chaud (50-60°C) avant émulsification. La solution aqueuse a ensuite été ajoutée au mélange huile/lécithine. Puis la solution bi-phasique a été émulsionnée pendant 5 minutes à 40 °C avec un sonificateur AV505 ® équipé d'une sonde conique de 3 mm de diamètre (Sonics, Newtown) réglé à 30 % de la puissance maximale. Les émulsions ainsi obtenues présentaient un diamètre de 35 nm (mesuré par un appareil Zetasizer Nano ® de chez Malvern Instrument), et une concentration en fluorophore de 400 µM.

A titre comparatif, une solution du fluorophore DiR à 10 mM dans le méthanol a également été préparée pour pourvoir observer l'effet de l'encapsulation sur la durée de vie de la fluorescence.

Les déclins de fluorescence du fluorophore libre en solution dans le méthanol ou encapsulé dans les nanoparticules lipidiques de type émulsions huile dans l'eau, en suspension dans du PBS (10 mM, pH 7,3) ont été mesurés sur une chaîne de mesure utilisant un laser Saphir-titane [Tsunami, Spectra-Physics,USA] (80Mhz, 100 femtoseconde), pompé par un laser continu néodyme-vanadate [Millennia Pro, Spectra-Physics, USA)] (532 nm, 5W) et accordable en longueur d'onde de 700 nm à 1000 nm. Selon le mode de fonctionnement de ce dispositif, le laser est injecté dans une fibre optique multimode, utilisée comme fibre excitatrice pour l'échantillon à étudier. Une deuxième fibre optique (de détection) collecte la fluorescence émise ou la diffusion laser *via* un système de filtrage. Le signal est mesuré par un tube photomultiplicateur [Hamamatsu, Japan] couplé à une carte de comptage TCSPC [Becker&Hickel, Germany]. Celle-ci est asservie par une partie prélevée (4 %) du signal laser (train d'impulsions) *via* une photodiode rapide (PD) [Becker&Hickel, Germany] avant l'injection dans la fibre optique.

Les mesures ont été faites en utilisant une longueur d'onde d'excitation pulsée de 740 nm. L'intensité de la fluorescence (en unités arbitraires) a été mesurée pour tracer des courbes reflétant le déclin de la fluorescence (intensité de la fluorescence (unités arbitraires) exprimée en fonction du temps (en ps)) ; courbes non représentées.

Les durées de vie de fluorescence ont ensuite été obtenues en utilisant le logiciel SPCImage (Becker Hickl GmbH) par un ajustement par un déclin mono-exponentiel (χᵣ² = 1.0) des courbes de déclin de fluorescence déconvoluées de la fonction de réponse instrumentale (IRF). Elles sont regroupées dans le tableau 2 ci-après :

**TABLEAU 2**

| **Durée de vie du Fluorophore DiR en solution dans le méthanol (en ps)** | **Durée de vie du fluorophore DiR dans la nanoémulsion (en ps)** |
|---|---|
| 800 | 1000 |

Ces résultats démontrent que l'encapsulation du fluorophore dans une nanoparticule lipidique de type nanoémulsion entraîne une augmentation de la durée de vie de fluorescence.

### EXEMPLE 6 : ETUDE DE L'EFFET DE L'ENCAPSULATION DU FLUOROPHORE DiD DANS DES NANOPARTICULES LIPIDIQUES SUR LE RENDEMENT QUANTIQUE DE FLUORESCENCE

Une nanoémulsion de même composition que celle décrite ci-dessus dans l'exemple 1 mais avec un taux de dopage en fluorophore DiD commercialisé par Invitrogen de 400 µM a été préparée.

Le rendement quantique de la fluorescence (Φ) est exprimé par l'équation suivante : Φ = (nombre de photons émis)/ (nombre de photons absorbés).

Il est courant de déterminer un rendement quantique par rapport à celui d'un étalon de référence par les techniques connues de l'homme de métier. Le rendement quantique de cette formulation a été calculé par comparaison avec un échantillon étalon de référence (Nile Blue perchlorate dans l'éthanol, commercialisé par Invitrogen) ayant une valeur du rendement quantique connue et égale à 0,27. Les mêmes mesures (réalisées à l'aide d'un spectrophotomètre Varian modèle Cary 300 et d'un spectrofluorimètre Perkin Elmer modèle LS 50) et calculs ont été réalisés pour une solution de fluorophore DiD dans le méthanol (MeOH).

Les résultats obtenus sont présentés dans le Tableau 3 ci-après :

**TABLEAU 3**

| **Fluorophore** | **DiD** | |
|---|---|---|
| | **En solution dans MeOH** | **Nanoémulsion** |
| **Max. Absorption (nm)** | 644 | 647 |
| **Emission (nm)** | 665 | 666 |
| **Rendement quantique** | 0.29 | 0.39 |

Ces résultats démontrent que l'encapsulation du fluorophore au coeur des gouttelettes d'huile d'une nanoémulsion huile-dans-eau permet d'augmenter le rendement quantique de fluorescence.

### EXEMPLE 7 : ENCAPSULATION DU DiD DANS DES NANOPARTICULES LIPIDIQUES AVEC DES COEURS HUILEUX DE DIFFÉRENTES COMPOSITIONS

Différentes formulations obtenues avec un taux de dopage en DiD de 400 µM mais avec un coeur lipidique de différentes compositions ont été préparées. Le mode de préparation de ces nanoémulsion est similaire à celui décrit dans l'exemple 1, mais la composition en a été modifiée, comme décrit dans les tableaux 4 et 5 ci-après. Pour chacune de ces formulations, la durée de vie de la fluorescence a été calculée selon la méthode expliquée ci-dessus à l'exemple 5. La longueur d'onde d'excitation était de 630 nm.

Ces formulations ont également été comparées avec une solution à 400 µM de DiD dans le méthanol (formulation A).

**TABLEAU 4 : Composition globale des nanoémulsions**

| **Quantités pour 5 g de nanoémulsion** | | | |
|---|---|---|---|
| | | **Masse (en mg)** | **%** |
| **Phase dispersée** | Huile totale (*) | 500 | 10 |
| **Tensioactifs** | Lécithine | 250 | 5 |
| | Myrj ® 53 | 827,5 | 16,55 |
| **Phase aqueuse** | Glycérol | 125 | 2,5 |
| | PBS | 3297,5 | 65,95 |

| | | | |
|---|---|---|---|
| (*) : correspond à la quantité totale de composants intervenant dans la phase huileuse dispersée et qui est détaillée dans le tableau 5 ci-après. | | | |

**TABLEAU 5 : Composition de la phase huileuse dispersée / Résultats**

| **Formulations** | **Composition de la phase huileuse dispersée (en % en poids) (pour 5 g de nanoémulsion)** | **Durée de vie de la fluorescence (ns)** |
|---|---|---|
| **A** | - | 1.15 ± 0.05 |
| **B** | Suppocire ® NC 100 % | 2.07 ± 0.05 |
| **C** | Huile de soja / Suppocire ® NC : 25/75 | 1.97 ± 0.05 |
| **D** | Huile de soja / Suppocire ® NC : 75/25 | 2.09 ± 0.05 |
| **E** | Huile de lin / Suppocire ® NC : 75/25 | 1.97 ± 0.05 |

On observe bien une augmentation de la durée de vie de fluorescence du DiD dans les formulations sous forme de nanoémulsion, et ce pour toutes les compositions lipidiques testées.

## Revendications

1. Emulsion fluorescente de type huile-dans-eau comprenant au moins une phase continue aqueuse et au moins un phase dispersée huileuse, ladite émulsion étant **caractérisée en ce que** :
- la phase dispersée est constituée de gouttelettes, d'au moins une huile biocompatible composée d'au moins une huile végétale ou animale et d'au moins une huile cristallisable riche en glycérides d'acides gras saturés en C₈-C₁₈ ayant la composition suivante :
C₈ : 0,1 à 0,9 %,
C₁₀ :0,1 à 0,9 %,
C₁₂ : 25 à 50 %,
C₁₄ : 10 à 24,9 %,
C₁₆: 10 à 24,9 %,
C₁₈ : 10 à 24,9 %,
lesdites gouttelettes ayant un de diamètre moyen supérieur ou égal à 10 nm et inférieur ou égal à 200 nm, et renfermant au moins un fluorophore lipophile absorbant et émettant à une longueur d'onde comprise entre 640 et 900 nm,
et **en ce que** lesdites gouttelettes sont stabilisées par une couche tensioactive située à la périphérie desdites gouttelettes, ladite couche comprenant, en mélange, au moins un tensioactif amphiphile et au moins un co-tensioactif de furtivité.

2. Emulsion selon la revendication 1, **caractérisée par le fait que** la taille des gouttelettes d'huile au sein de l'émulsion est comprise entre 10 et 80 nm inclusivement,

3. Emulsion selon la revendication 1 ou 2, **caractérisée par le fait que** l'huile végétale est l'huile de soja.

4. Emulsion selon l'une quelconque des revendications précédentes, **caractérisée par le fait que** le rapport pondéral huile végétale ou animale / huile cristallisable riche en glycérides varie entre 10/90 et 90/10 inclusivement.

5. Emulsion selon l'une quelconque des revendications précédentes, **caractérisée par le fait que** les tensioactifs amphiphiles sont choisis parmi les composés dont la partie lipophile comprend une chaîne saturée ou insaturée, linéaire ou ramifiée, ayant de 8 à 30 atomes de carbone.

6. Emulsion selon l'une quelconque des revendications précédentes, **caractérisée par le fait que** les tensioactifs sont choisis parmi les phospholipides, les cholestérols, les lysolipides, les sphingomyélines, les tocophérols, les glucolipides, stéarylamines, les cardiolipines d'origine naturelle ou synthétique ; les molécules composées d'un acide gras couplé à un groupement hydrophile par une fonction éther ou ester ; les lipides polymérisés ; les lipides conjugués à de courtes chaînes d'oxyde de polyéthylène (PEG) ; les esters de sucre ; lesdits tensioactifs pouvant être utilisés seuls ou en mélanges.

7. Emulsion selon la revendication 6, **caractérisée par le fait que** les tensioactifs sont choisis parmi la lécithine de soja, les phospholipides et le cholestérol.

8. Emulsion selon l'une quelconque des revendications précédentes, **caractérisée par le fait que** les fluorophores lipophiles sont choisis parmi les analogues d'acides gras, les sphingolipides, les stéroïdes, les lipo-polysaccharides et les phospholipides fonctionnalisés par un groupement absorbant et émettant entre 640 et 900 nm, et leurs dérivés amphiphiles.

9. Emulsion selon la revendication 8, **caractérisée par le fait que** les fluorophores sont choisis parmi les dérivés amphiphiles de dialkylcarbocyanines.

10. Emulsion selon l'une quelconque des revendications précédentes, **caractérisée par le fait que** le ou les co-tensioactifs de furtivité sont choisis parmi les molécules amphiphiles dont la partie hydrophile est totalement ou en partie composée d'une chaîne d'oxyde de polyéthylène (PEO) dans laquelle le nombre de motifs PEO varie de 2 à 500 et les polysaccharides.

11. Emulsion selon la revendication 10, **caractérisée par le fait que** le ou les co-tensioactifs de furtivité sont choisis parmi les composés conjugués polyéthylèneglycol/phosphatidyl-éthanolamine (PEG-PE), les éthers d'acide gras et de polyéthylèneglycol, les esters d'acide gras et de polyéthylèneglycol et les copolymères blocs d'oxyde d'éthylène et d'oxyde de propylène.

12. Emulsion selon l'une quelconque des revendications précédentes, **caractérisée par le fait que** la couche tensioactive située à la périphérie des gouttelettes d'huile de l'émulsion comprend en outre au moins un agent de ciblage d'une activité biologique d'intérêt, ledit agent de ciblage étant constitué d'un co-tensioactif de greffage amphiphile dont la partie hydrophile est liée, de façon covalente, à un ligand biologique.

13. Emulsion selon la revendication 12, **caractérisée par le fait que** lesdits agents de ciblage sont choisis parmi les composés de formule (1) suivante : 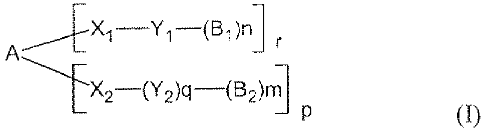 dans laquelle :
- A est la partie lipophile d'un co-tensioactif de greffage amphiphile (CoTA),
- X₁ et X₂, identiques ou différents, constituent la partie hydrophile dudit co-tensioactif CoTA et sont composés d'un bras espaceur flexible choisi parmi les chaînes carbonées linéaires ou ramifiées, saturées ou insaturées, éventuellement substituées, interrompues et/ou terminées par un ou plusieurs hétéroatomes choisis parmi N, O, P et S, et/ou par un ou plusieurs groupements choisis parmi les radicaux alkyle en C₁-C₄, alcoxy en C₁-C₄, aryle ou par une ou plusieurs fonctions choisies parmi les fonctions éther, ester, amide, carbonyle, carbamate, urée, thiourée et disulfure ;
- Y₁ et Y₂, identiques ou différents, sont choisis parmi les groupements chimiques aptes à relier X₁ et B₁, respectivement X₂ et B₂, par des liaisons covalentes ;
- B₁ et B₂, identiques ou différents, sont des ligands biologiques dont l'une des extrémités est engagée dans la liaison covalente formée avec X₁, respectivement X₂ ;
- n est un nombre entier compris entre 1 et 20 inclusivement ;
- q est un nombre entier égal à 0 ou 1 ;
- m est un nombre entier compris entre 0 et 20 inclusivement, étant entendu que m = 0 lorsque q = 0 ;
- p est un nombre entier compris entre 0 et 10 inclusivement ; et
- R est un nombre entier compris entre 0 et 10 inclusivement.

14. Emulsion selon la revendication 13, **caractérisée par le fait que** la partie hydrophile du CoTA constituant les bras espaceurs X₁ et X₂ des composés de formule (I) est choisie parmi les chaînes constituées de motifs polyoxyéthylène ou dextrane.

15. Emulsion selon l'une quelconque des revendications 12 à 14, **caractérisée par le fait que** les ligands biologiques utilisables à titre d'unité B₁/B₂ des agents de ciblage de formule (I) sont choisis parmi :
i) les peptides, leurs dérivés et leurs analogues, les protéines, les anti-corps, leurs dérivés et leurs analogues ; les monosaccharides, les oligosaccharides, les polysaccharides, leurs dérivés et leurs analogues ; les oligonucléotides, l'ADN, leurs dérivés et leurs analogues ; les molécules organiques, les complexes organométalliques dont l'activité de ciblage est due à la reconnaissance moléculaire de ces ligands par des récepteurs sur-exprimés à la surface des cellules de la zone d'intérêt ;
ii) les ligands biologiques marqueurs d'une activité enzymatique et les ligands comportant un pont disulfure séparant le label d'un inhibiteur de sa fluorescence.

16. Emulsion selon l'une quelconque des revendications précédentes, **caractérisée par le fait que** la phase continue de l'émulsion est une phase aqueuse constituée d'eau et/ou d'un tampon physiologiquement acceptable ou d'une solution de chlorure de sodium.

17. Réactif de diagnostic d'une activité biologique d'intérêt, **caractérisé par le fait qu'**il comprend au moins une émulsion fluorescente telle que définie à l'une quelconque des revendications précédentes.

18. Réactif selon la revendication 17, **caractérisé par le fait qu'**il s'agit d'un réactif de diagnostic *in vivo.*

19. Utilisation d'au moins une émulsion fluorescente telle que définie à l'une quelconque des revendications 1 à 16, pour la préparation d'un réactif de diagnostic d'une activité biologique d'intérêt *in vivo* par imagerie de fluorescence et/ou pour l'aide au développement et à l'optimisation d'outils thérapeutiques.

20. Utilisation d'au moins une émulsion fluorescente telle que définie à l'une quelconque des revendications 1 à 16, pour la préparation d'un réactif de diagnostic d'une activité biologique d'intérêt *in vivo* par imagerie de fluorescence résolue dans le temps.

## Patentansprüche

1. Fluoreszierende Emulsion vom Typ Öl-in-Wasser, umfassend mindestens eine kontinuierliche wässrige Phase und mindestens eine dispergierte ölige Phase, wobei die Emulsion **dadurch gekennzeichnet ist, dass**:
- die dispergierte Phase von Tröpfchen mindestens eines biokompatiblen Öls gebildet wird, das aus mindestens einem pflanzlichen oder tierischen Öl und mindestens einem kristallisierbaren Öl besteht, welches reich an gesättigten C₈-C₁₈ Fettsäureglyceriden ist, die die folgende Zusammensetzung aufweisen:
C₈: 0,1 bis 0,9 %,
C₁₀: 0,1 bis 0,9 %,
C₁₂: 25 bis 50 %,
C₁₄: 10 bis 24,9 %,
C₁₆: 10 bis 24,9 %,
C₁₈: 10 bis 24,9 %,
wobei die Tröpfchen einen mittleren Durchmesser von größer als oder gleich 10 nm und kleiner als oder gleich 200 nm aufweisen und mindestens ein lipophiles Fluorophor umschließen, welches eine Wellenlänge im Bereich zwischen 640 und 900 nm absorbiert und emittiert,
und dadurch, dass die Tröpfchen von einer am Umfang der Tröpfchen liegenden Tensidschicht stabilisiert werden, wobei die Schicht mindestens ein amphiphiles Tensid und mindestens ein tarnendes Cotensid in Mischung umfasst.

2. Emulsion nach Anspruch 1, **gekennzeichnet durch** die Tatsache, dass die Größe der Öltröpfchen innerhalb der Emulsion im Bereich zwischen einschließlich 10 und 80 nm beträgt.

3. Emulsion nach Anspruch 1 oder 2, **gekennzeichnet durch** die Tatsache, dass das pflanzliche Öl Sojaöl ist.

4. Emulsion nach einem der vorstehenden Ansprüche, **gekennzeichnet durch** die Tatsache, dass das Gewichtsverhältnis pflanzliches oder tierisches Öl / kristallisierbares glyceridreiches Öl zwischen einschließlich 10/90 und 90/10 variiert.

5. Emulsion nach einem der vorstehenden Ansprüche, **gekennzeichnet durch** die Tatsache, dass die amphiphilen Tenside ausgewählt sind aus den Verbindungen, deren lipophiler Teil eine gesättigte oder ungesättigte, geradkettige oder verzweigte Kette umfasst, die 8 bis 30 Kohlenstoffatome aufweist.

6. Emulsion nach einem der vorstehenden Ansprüche, **gekennzeichnet durch** die Tatsache, dass die Tenside ausgewählt sind aus den Phospholipiden, den Cholesterolen, den Lysolipiden, den Sphingomyelinen, den Tocopherolen, den Glykolipiden, Stearylaminen, den Cardiolipinen natürlichen oder synthetischen Ursprungs; den Molekülen, die aus einer über eine Ether- oder Esterfunktion an eine hydrophile Gruppe gekoppelten Fettsäure bestehen; den polymerisierten Lipiden; den Lipiden, die an kurze Polyethylenoxid- (PEG) Ketten konjugiert sind; den Zuckerestern; wobei die Tenside alleine oder in Mischungen verwendet werden können.

7. Emulsion nach Anspruch 6, **gekennzeichnet durch** die Tatsache, dass die Tenside ausgewählt sind aus Sojalecithin, den Phospholipiden und Cholesterol.

8. Emulsion nach einem der vorstehenden Ansprüche, **gekennzeichnet durch** die Tatsache, dass die lipophilen Fluorophore ausgewählt sind aus den Fettsäure-Analoga, den Sphingolipiden, den Steroiden, den Lipopolysacchariden und den Phospholipiden, die über eine Gruppe funktionalisiert sind, welche zwischen 640 und 900 nm absorbiert und emittiert, und deren amphiphilen Derivaten.

9. Emulsion nach Anspruch 8, **gekennzeichnet durch** die Tatsache, dass die Fluorophore ausgewählt sind aus den amphiphilen Dialkylcarbocyanin-Derivaten.

10. Emulsion nach einem der vorstehenden Ansprüche, **gekennzeichnet durch** die Tatsache, dass das oder die tarnenden Cotenside aus den amphiphilen Molekülen ausgewählt sind, deren hydrophiler Teil vollständig oder zum Teil aus einer Polyethylenoxid- (PEO) Kette, in der die Anzahl an PEO-Motiven von 2 bis 500 variiert, und den Polysacchariden besteht.

11. Emulsion nach Anspruch 10, **gekennzeichnet durch** die Tatsache, dass das oder die tarnenden Cotenside ausgewählt sind aus den konjugierten Polyethylenglykol/Phosphatidyl-Ethanolamin- (PEG-PE) Verbindungen, den Fettsäure- und Polyethylenglykol-Ethern, den Fettsäure- und Polyethylenglykol-Estern und den Ethylenoxid- und Propylenoxid-Blockcopolymeren.

12. Emulsion nach einem der vorstehenden Ansprüche, **gekennzeichnet durch** die Tatsache, dass die am Umfang der Öltröpfchen der Emulsion liegende Tensidschicht weiter mindestens ein Agens zur zielgerichteten Ansteuerung einer interessierenden biologischen Aktivität umfasst, wobei das zielgerichtete Agens von einem amphiphilen Pfropf-Cotensid gebildet wird, dessen hydrophiler Teil in kovalenter Weise an einen biologischen Liganden gebunden ist.

13. Emulsion nach Anspruch 12, **gekennzeichnet durch** die Tatsache, dass die zielgerichteten Agentien ausgewählt sind aus den Verbindungen folgender Formel (I) : 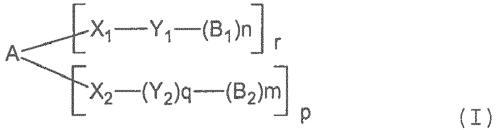 wobei:
- A der lipophile Teil eines amphiphilen Pfropf-Cotensids (CoTA) ist,
- X₁ und X₂, identisch oder verschieden, den hydrophilen Teil des CoTA-Cotensids bilden und aus einem flexiblen Spacerarm bestehen, ausgewählt aus den geradkettigen oder verzweigten, gesättigten oder ungesättigten, gegebenenfalls mit einem oder mehreren Heteroatomen, ausgewählt aus N, O, P und S, und/oder mit einer oder mehreren Gruppen, ausgewählt aus den C₁-C₄ Alkyl-, C₁-C₄ Alkoxy-, Arylresten, oder mit einer oder mehreren Funktionen, ausgewählt aus den Ether-, Ester-, Amid-, Carbonyl-, Carbamat-, Harnstoff-, Thioharnstoff- und Disulfidfunktionen substituierten, unterbrochenen und/oder terminierten kohlenstoffhaltigen Ketten bestehen;
- Y₁ und Y₂, identisch oder verschieden, ausgewählt sind aus den chemischen Gruppen, die in der Lage sind, X₁ und B₁, beziehungsweise X₂ und B₂ über kovalente Bindungen zu verbinden;
- B₁ und B₂, identisch oder verschieden, biologische Liganden sind, von denen eines der Enden in die mit X₁ beziehungsweise X₂ gebildete kovalente Bindung eingebunden ist;
- n eine ganze Zahl im Bereich zwischen einschließlich 1 und 20 ist;
- q eine ganze Zahl von gleich 0 oder 1 ist;
- m eine ganze Zahl im Bereich zwischen einschließlich 0 und 20 ist, wobei davon ausgegangen wird, dass m = 0 wenn q = 0;
- p eine ganze Zahl im Bereich zwischen einschließlich 0 und 10 ist; und
- R eine ganze Zahl im Bereich zwischen einschließlich 0 und 10 ist.

14. Emulsion nach Anspruch 13, **gekennzeichnet durch** die Tatsache, dass der hydrophile Teil des CoTA, welcher die Spacerarme X₁ und X₂ der Verbindungen der Formel (I) bildet, ausgewählt ist aus den Ketten, die aus Polyoxyethylen- oder Dextran-Motiven gebildet sind.

15. Emulsion nach einem der Ansprüche 12 bis 14, **gekennzeichnet durch** die Tatsache, dass die biologischen Liganden, die als Einheit B₁/B₂ der zielgerichteten Agentien der Formel (I) verwendbar sind, ausgewählt sind aus:
i) den Peptiden, deren Derivaten und deren Analoga, den Proteinen, den Antikörpern, deren Derivaten und deren Analoga; den Monosacchariden, den Oligosacchariden, den Polysacchariden, deren Derivaten und deren Analoga; den Oligonukleotiden, DNA, deren Derivaten und deren Analoga; den organischen Molekülen, den metallorganischen Komplexen, deren zielgerichtete Aktivität auf die molekulare Erkennung dieser Liganden durch Rezeptoren zurückgeht, die an der Oberfläche der Zellen des interessierenden Bereichs überexprimiert sind;
ii) den biologischen Liganden, die eine enzymatische Aktivität markieren, und den Liganden, die eine Disulfidbrücke umfassen, welche die Markierung eines Inhibitors von seiner Fluoreszenz trennt.

16. Emulsion nach einem der vorstehenden Ansprüche, **gekennzeichnet durch** die Tatsache, dass die kontinuierliche Phase der Emulsion eine wässrige Phase ist, die von Wasser und/oder einem physiologisch verträglichen Puffer oder einer Natriumchloridlösung gebildet wird.

17. Reagens zur Diagnose einer interessierenden biologischen Aktivität, **gekennzeichnet durch** die Tatsache, dass es mindestens eine fluoreszierende Emulsion wie in einem der vorstehenden Ansprüche definiert umfasst.

18. Reagens nach Anspruch 17, **gekennzeichnet durch** die Tatsache, dass es sich um ein *In-vivo-*Diagnosereagens handelt.

19. Verwendung von mindestens einer fluoreszierenden Emulsion wie in einem der Ansprüche 1 bis 16 definiert, zur Herstellung eines Reagens zur Diagnose einer interessierenden biologischen Aktivität *in vivo* durch Fluoreszenzbildgebung und/oder zur Unterstützung der Entwicklung und der Optimierung von therapeutischen Instrumenten.

20. Verwendung von mindestens einer fluoreszierenden Emulsion wie in einem der Ansprüche 1 bis 16 definiert, zur Herstellung eines Reagens zur Diagnose einer interessierenden biologischen Aktivität *in vivo* durch zeitaufgelöste Fluoreszenzbildgebung.

## Claims

1. Fluorescent emulsion of the oil-in-water type comprising at least one continuous aqueous phase and at least one oily dispersed phase, said emulsion being **characterised in that**:
- the dispersed phase is made of droplets, of at least one biocompatible oil made of at least one plant or animal oil and of at least one crystallisable oil rich in C₈-C₁₈ saturated fatty acid glycerides having the following composition:
C₈: 0.1 to 0.9%,
C₁₀: 0.1 to 0.9%,
C₁₂: 25 to 50%,
C₁₄: 10 to 24.9%,
C₁₆: 10 to 24.9%,
C₁₈: 10 to 24.9%,
said droplets having an average diameter greater than or equal to 10 nm and less than or equal to 200 nm, and containing at least one lipophilic fluorophore absorbing and emitting at a wavelength between 640 and 900 nm,
and **in that** said droplets are stabilised by a surfactant layer located at the periphery of said droplets, said layer comprising, in a mixture, at least one amphiphilic surfactant and at least one stealth co-surfactant.

2. Emulsion according to claim 1, **characterised by** the fact that the size of the oil droplets in the emulsion is between 10 and 80 nm inclusively.

3. Emulsion according to claim 1 or 2, **characterised by** the fact that the plant oil is soy oil.

4. Emulsion according to anyone of the preceding claims, **characterised by** the fact that the plant or animal oil / crystallisable oil rich in glycerides weight ratio varies between 10/90 and 90/10 inclusively.

5. Emulsion according to anyone of the preceding claims, **characterised by** the fact that the amphiphilic surfactants are chosen from compounds of which the lipophilic portion comprises a linear or branched, saturated or unsaturated chain, having from 8 to 30 carbon atoms.

6. Emulsion according to anyone of the preceding claims, **characterised by** the fact that the surfactants are chosen from phospholipids, cholesterols, lysolipids, sphingomyelins, tocopherols, glucolipids, stearylamines, cardiolipins of natural or synthetic origin; molecules made of a fatty acid coupled to a hydrophilic group by an ether or ester function; polymerised lipids; lipids conjugated with short chains of polyethylene oxide (PEG); esters of sugar; said surfactants being able to be used alone or in mixtures.

7. Emulsion according to claim 6, **characterised by** the fact that the surfactants are chosen from soya lecithin, phospholipids and cholesterol.

8. Emulsion according to anyone of the preceding claims, **characterised by** the fact that the lipophilic fluorophores are chosen from fatty acid analogues, sphingolipids, steroids, lipo-polysaccharides and phospholipids functionalised by a group absorbing and emitting between 640 and 900 nm, and the amphiphilic derivatives thereof.

9. Emulsion according to claim 8, **characterised by** the fact that the fluorophores are chosen from amphiphilic derivatives of dialkylcarbocyanins.

10. Emulsion according to anyone of the preceding claims, **characterised by** the fact that the stealth co-surfactant or co-surfactants are chosen from amphiphilic molecules of which the hydrophilic portion is entirely or partially made of a polyethylene oxide (PEO) chain in which the number of PEO units varies from 2 to 500 and polysaccharides.

11. Emulsion according to claim 10, **characterised by** the fact that the stealth co-surfactant or co-surfactants are chosen from polyethylene glycolphosphatidylethanolamine (PEG-PE) conjugated compounds, ethers of fatty acid and of polyethylene glycol, esters of fatty acid and of polyethylene glycol and block copolymers of ethylene oxide and of propylene oxide.

12. Emulsion according to anyone of the preceding claims, **characterised by** the fact that the surfactant layer located at the periphery of the oil droplets of the emulsion further comprises at least one agent for targeting a biological activity of interest, said targeting agent being made of an amphiphilic grafting co-surfactant of which the hydrophilic portion is covalently bonded to a biological ligand.

13. Emulsion according to claim 12, **characterised by** the fact that said targeting agents are chosen from compounds of the following formula (I): 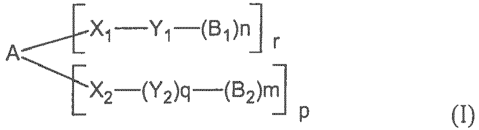 wherein:
- A is the lipophilic portion of an amphiphilic grafting co-surfactant (CoTA),
- X₁ and X₂, identical or different, constitute the hydrophilic part of said co-surfactant CoTA and are made of a flexible spacer arm chosen from saturated or unsaturated, linear or branched carbon-based chains optionally substituted, interrupted and/or terminated with one or several heteroatoms chosen from N, O, P and S, and/or with one or more groups chosen from C₁-C₄ alkyl, C₁-C₄ alkoxy, and aryl radicals or with one or several functions chosen from ether, ester, amide, carbonyl, carbamate, urea, thiourea and disulfide functions;
- Y₁ and Y₂, identical or different, are chosen from the chemical groups able to link X₁ and B₁, respectively X₂ and B₂, by covalent bonds;
- B₁ and B₂, identical or different, are biological ligands of which one of the ends is involved in the covalent bond formed with X₁, respectively X₂;
- n is an integer between 1 and 20 inclusively;
- q is an integer equal to 0 or 1;
- m is an integer between 0 and 20 inclusively, provided that m = 0 when q = 0;
- p is an integer between 0 and 10 inclusively; and
- R is an integer between 0 and 10 inclusively.

14. Emulsion according to claim 13, **characterised by** the fact that the hydrophilic portion of the CoTA comprising the spacer arms X₁ and X₂ of the compounds of formula (I) is chosen from chains comprised of polyoxyethylene or dextran units.

15. Emulsion according to anyone of claims 12 to 14, **characterised by** the fact that the biological ligands that can be used as a unit B₁/B₂ of the targeting agents of formula (I) are chosen from:
i) peptides, the derivatives and analogues thereof, proteins, antibodies, the derivatives and analogues thereof; monosaccharides, oligosaccharides, polysaccharides, the derivatives and analogues thereof; oligonucleotides, DNA, the derivatives and analogues thereof; organic molecules, and organometallic complexes, the targeting activity of which is due to the molecular recognition of these ligands by receptors overexpressed at the surface of the cells of the region of interest;
ii) biological ligands which are markers for an enzymatic activity and ligands comprising a disulfide bridge separating the label from an inhibitor of its fluorescence.

16. Emulsion according to anyone of the preceding claims, **characterised by** the fact that the continuous phase of the emulsion is an aqueous phase made of water and/or a physiologically acceptable buffer or of a sodium chloride solution.

17. Diagnostic reagent for diagnosing a biological activity of interest, **characterised by** the fact that it comprises at least one fluorescent emulsion such as defined in any of the preceding claims.

18. Reagent according to claim 17, **characterised by** the fact that it is an *in vivo* diagnostic reagent.

19. Use of at least one fluorescent emulsion such as defined in anyone of claims 1 to 16, for the preparation of a diagnostic reagent of an *in vivo* biological activity of interest by fluorescence imaging and/or for assistance in developing and in optimising therapeutic tools.

20. Use of at least one fluorescent emulsion such as defined in anyone of claims 1 to 16, for the preparation of a diagnostic reagent of an *in vivo* biological activity of interest by time resolved fluorescence imaging.
